# EUROPEAN PATENT APPLICATION

(11) **EP 1 870 416 A1**
(43) Date of publication of application: **26.12.2007**
(21) Application number: 06090115.4
(22) Date of filing: 21.06.2006
(51) Int. Cl.: C07D 513/08, A61K 31/529, A61P 33/00

(54) **Sulphonamido-macrocycles as tie2 inhibitors**

(71) Applicant: Bayer Schering Pharma Aktiengesellschaft, 13353 Berlin (DE)
(72) Inventor: Hartung, Ingo, 13469 Berlin (DE); Briem, Hans, 28279 Bremen (DE); Kettschau, Georg, 13187 Berlin (DE); Thierauch, Karl-Heinz, 14169 Berlin (DE); Lücking, Ulrich, 10245 Berlin (DE); Bömer, Ulf, 16548 Glienicke/Nordbahn (DE); Shäfer, Martina, 13187 Berlin (DE); Lienau, Philp, 10997 Berlin (DE)

(57) **Abstract**

The invention relates to sulfonamido-macrocycles according to the general formula (I): 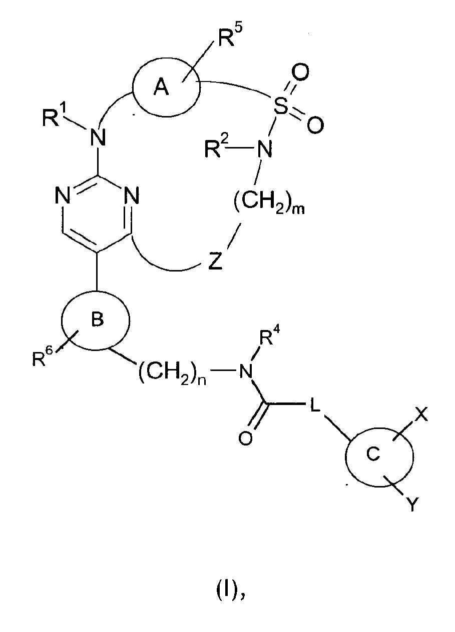 in which R¹, R², R⁴, R⁵, R⁶, A, B, C, L, X, Y, Z, n, and m are as defined in the claims, and salts thereof, to pharmaceutical compositions comprising said sulfonamido-macrocycles, to methods of preparing said sulfonamido-macrocycles as well as the use thereof for manufacturing a pharmaceutical composition for the treatment of diseases of dysregulated vascular growth or of diseases which are accompanied with dysregulated vascular growth, wherein the compounds effectively interfere with Tie2 signalling.

## Description

The present invention relates to sulfonamido-macrocycles of general formula (I) and salts thereof, to pharmaceutical compositions comprising said sulfonamido-macrocycles, to methods of preparing said sulfonamido-macrocycles, as well as to uses thereof.

Dysregulated vascular growth plays a critical role in a variety of inflammatory diseases, in particular psoriasis, delayed type hypersensitivity, contact dermatitis, asthma, multiple sclerosis, restenosis, rheumatoid arthritis and inflammatory bowl disease. Aberrant vascular growth is also involved in neovascular ocular diseases such as age-related macular degeneration and diabetic retinopathy. Additionally, sustained vascular growth is accepted as one hallmark of cancer development (Hanahan, D.; Weinberg, R. A. Cell 2000, 100, 57). While tumours initially grow either as an avascular mass or by co-opting existing host vessels, growth beyond a few mm³ in size is depending on the induction of vessel neogrowth in order to sufficiently provide the tumour with oxygen and nutrients. Induction of angiogenesis is a prerequisite that the tumour surpasses a certain size (the so called angiogenic switch). An intricate signalling interaction network between cancer cells and the tumour microenvironment triggers the induction of vessel growth from existing vasculature. The dependence of tumours on neovascularization has led to a new treatment paradigm in cancer therapy (Ferrara et al. Nature 2005, 438, 967; Carmeliet Nature 2005, 438, 932). Blocking tumour neovascularization by small molecule or antibody-mediated inhibition of relevant signal transduction pathways holds a great promise for extending currently available therapy options.

The development of the cardiovascular system involves two basic stages. In the initial vasculogenesis stage, which only occurs during embryonal development, angioblasts differentiate into endothelial cells which subsequently form a primitive vessel network. The subsequent stage, termed angiogenesis, involves the remodelling of the initial vasculature and sprouting of new vessels (Risau, W. Nature 1997, 386, 671; Jain, R. K. Nat. Med. 2003, 9, 685). Physiologically, angiogenesis occurs in wound healing, muscle growth, the female cycle and in the above mentioned disease states.

It has been found that receptor tyrosine kinases of the vascular endothelial growth factor (VEGF) family and the Tie (tyrosine kinase with immunoglobulin and epidermal growth factor homology domain) receptor tyrosine kinases are essential for both developmental and disease-associated angiogenesis (Ferrara et al Nat. Med. 2003, 9, 669; Dumont et al. Genes Dev. 1994, 8, 1897; Sato et al. Nature 1995, 376, 70).

In adults the Tie2 receptor tyrosine kinase is selectively expressed on endothelial cells (EC) of the adult vasculature (Schlaeger et al. Proc. Not. Acad. Sci. USA 1997, 94, 3058). Immunohistochemical analysis demonstrated the expression of Tie2 in adult rat tissues undergoing angiogenesis. During ovarian folliculogenesis, Tie2 is expressed in neovessels of the developing corpus luteum. Four endogeneous ligands - angiopoietins 1 to 4 - have been identified for the type 1 transmembrane Tie2 (also named Tek) receptor, while no ligands have been identified so far for the Tie1 receptor. Binding of the extracellular Tie2 domain to the C-terminal fibrinogen-like domains of the various angiopoietins leads to significantly different cellular effects. In addition, heterodimerizations between Tie1 and Tie2 receptors have been postulated to influence ligand binding.

Binding of Ang1 to Tie2 expressed on EC induces receptor cross-phosphorylation and kinase activation thus triggering various intracellular signalling pathways. The intracellular C-terminal tail of the Tie2 protein plays a crucial role in Tie2 signalling (Shewchuk et al. Structure 2000, 8, 1105). Upon ligand binding, a conformational change is induced which removes the C-tail out of its inhibitory conformation thus allowing kinase activation by cross-phoshorylation of various Tyr residues in the C-tail, which subsequently function as docking sites for phosphotyrosine-binding (PTB) site possessing down-stream mediators. Cellular effects initiated by Ang1 activation of Tie2 include inhibition of EC apoptosis, stimulation of EC migration and blood vessel reorganization, suppression of inflammatory gene expression and suppression of vascular permeability (Brindle et al. Circ. Res. 2006, 98, 1014). In contrast to VEGF-VEGFR signalling in EC, Ang1 activation of Tie2 does not stimulate EC proliferation in the majority of published assay settings.

The anti-apoptotic effect of Tie2 signalling was shown to be mediated mainly by the P13K-Akt signalling axis which is activated by binding of the regulatory p85 subunit of P13K to Y1102 in the Tie2 C-tail (DeBusk et al. Exp. Cell. Res. 2004, 298, 167; Papapetropoulos et al. J. Biol. Chem. 2000, 275, 9102; Kim et al. Circ. Res. 2000, 86, 24). In contrast, the chemotactic response downstream of the activated Tie2 receptor requires crosstalk between P13K and the adaptor protein Dok-R. Membrane localization of Dok-R via binding of its plekstrin homology (PH) domain to PI3K and simultaneous binding to Y1108 in the Tie2 C-tail via its PTB domain leads to Dok-R phoshorylation and downstream signalling via Nck and Pak-1 (Jones et al. Mol. Cell Biol. 2003, 23, 2658; Master et al. EMBO J. 2001, 20, 5919). PI3K-mediated recruitment of the adaptor protein ShcA to Y1102 of the Tie2 C-tail is also believed to induce cellular sprouting and motility effects involving activation of endothelial nitric oxide synthase (eNOS), focal adhesion kinase (FAK) and the GTPases RhoA and Rac1. Other downstream mediators of Tie2 signalling include the adaptor protein Grb2, which mediates Erk1/2 stimulation, and the SHP-2 phosphatase.

In conclusion, basal activation of the Tie2 pathway by Ang1 is believed to maintain quiescence and integrity of the endothelium of the adult vasculature by providing a cell survival signal for ECs and by maintaining the integrity of the EC lining of blood vessels (Peters et al. Recent Prog. Horm. Res. 2004, 59, 51).

In contrast to Ang1, Ang2 is not able to activate Tie2 on EC unless Ang2 is present in high concentration or for prolonged periods. However, Ang2 functions as a Tie2 agonist in non-endothelial cells transfected with Tie2. The structural basis for this context-dependence of the Ang2-Tie2 interaction is to date not understood.

In endothelial cells, however, Ang2 functions as Tie2 antagonist and thus blocks the agonistic activity of Ang1 (Maisonpierre et al. Science 1997, 277, 55). Ang2 binding to Tie2 prevents Ang1-mediated Tie2 activation which leads to vessel destabilization and results in vessel regression in the absence of pro-angiogenic stimuli such as VEGF. While Ang1 is widely expressed by periendothelial cells in quiescent vasculature such as pericytes or smooth muscle cells, Ang2 expression occurs in areas of ongoing angiogenesis. Ang2 can be stored in Weibel-Palade bodies in the cytoplasm of EC allowing for a quick vascular response upon stimulation.

Ang1 and Ang2 are expressed in the corpus luteum, with Ang2 localizing to the leading edge of proliferating vessels and Ang1 localizing diffusively behind the leading edge. Ang2 expression is *inter alia* initiated by hypoxia (Pichiule et al. J. Biol. Chem. 2004, 279, 12171). Ang2 is upregulated in the tumour vasculature and represents one of the earliest tumour markers. In the hypoxic tumour tissue, Ang2 expression induces vessel permeability and - in the presence of e.g. pro-angiogenic VEGF - triggers angiogenesis. After VEGF mediated EC proliferation and vessel sprouting maturation of the newly formed vessels again necessitates Tie2 activation by Ang1. Therefore, a subtle balancing of Tie2 activity plays a pivotal role in the early as well as late stages of neovascularization. These observations render the Tie2 RTK an attractive target for anti-angiogenesis therapy in diseases caused by or associated with dysregulated vascular growth. However, it remains to be shown if targeting the Tie2 pathway alone will be sufficient to achieve efficacious blockade of neovascularization. In certain diseases or disease subtypes it might be necessary or more efficacious to block several angiogenesis-relevant signalling pathways simultaneously.

Various theories have been discussed to explain the differential effects of Ang1 and Ang2 on Tie2 downstream signalling events. Binding of Ang1 and Ang2 in a structurally different manner to the Tie2 ectodomain could induce ligand-specific conformational changes of the intracellular kinase domain explaining different cellular effects. Mutational studies however point toward similar binding sites of Ang1 and Ang2. In contrast, various publications have focussed on different oligomerization states of Ang1 vs. Ang2 as basis for different receptor multimerization states upon ligand binding. Only Ang1 present in its tetramer or higher-order structure initiates Tie2 activation in EC while Ang2 was reported to exist as a homodimer in its native state (Kim et al. J. Biol. Chem. 2005, 280, 20126; Davis et al. Nat. Struc. Biol. 2003, 10, 38; Barton et al. Structure 2005, 13, 825). Finally, specific interactions of Ang1 or Ang2 with additional cell-specific co-receptors could be responsible for the different cellular effects of Ang1 vs. Ang2 binding to Tie2. Interaction of Ang1 with integrin α5β1 has been reported to be essential for certain cellular effects (Carlson et al. J. Biol. Chem. 2001, 276, 26516; Dallabrida et al. Circ. Res. 2005, 96, e8). Integrin α5β1 associates constitutively with Tie2 and increases the receptor's binding affinity for Ang1 resulting in initiation of downstream signalling at lower Ang1 effector concentrations in situations where integrin α5β1 is present. The recently solved crystal structure of the Tie2-Ang2 complex suggests however that neither the oligomerization state nor a different binding mode causes the opposing cellular effects (Barton et al. Nat. Struc. Mol. Biol. 2006, advance online publication).

Ang1-Tie2 signalling plays also a role in the development of the lymphatic system and in lymphatic maintenance and sprouting (Tammela *et al. Blood* **2005,** *105,* 4642). An intimate cross-talk between Tie2 and VEGFR-3 signalling in lymphangiogenesis seems to equal the Tie2-KDR cross-talk in blood vessel angiogenesis.

A multitude of studies have underscored the functional significance of Tie2 signalling in the development and maintenance of the vasculature. Disruption of Tie2 function in Tie2^{-/-} transgenic mice leads to early embryonic lethality between days 9.5 and 12.5 as a consequence of vascular abnormalities. Tie2^{-/-} embryos fail to develop the normal vessel hierachy suggesting a failure of vascular branching and differentiation. The heart and vessels in Tie2^{-/-} embryos show a decreased lining of EC and a loosened interaction between EC and underlying pericyte/smooth muscle cell matrix. Mice lacking functional Ang1 expression and mice overexpressing Ang2 display a phenotype reminiscent of the phenotype of Tie2^{-/-} mice (Suri et al. Cell 1996, 87, 1171). Ang2^{-/-} mice have profound defects in the growth and patterning of lymphatic vasculature and fail to remodel and regress the hyaloid vasculature of the neonatal lens (Gale et al. Dev. Cell 2002, 3, 411). Ang1 rescued the lymphatic defects, but not the vascular remodelling defects. Therefore, Ang2 might function as a Tie2 antagonist in blood vasculature but as a Tie2 agonist in developing lymph vasculature suggesting redundant roles of Ang1 and Ang2 in lymphatic development.

Aberrant activation of the Tie2 pathway is involved in various pathological settings. Activating Tie2 mutations leading to increased ligand-dependent and ligand-independent Tie2 kinase activity cause inherited venous malformations (Vikkula et al. Cell 1996, 87, 1181). Increased Ang1 mRNA and protein levels as well as increased Tie2 activation have been reported in patients with pulmonary hypertension (PH). Increased pulmonary arterial pressure in PH patients results from increased coverage of pulmonary arterioles with smooth muscle cells (Sullivan et al. Proc. Natl. Acad. Sci. USA 2003, 100, 12331). In chronic inflammatory diseases, like in psoriasis, Tie2 and the ligands Ang1 and Ang2 are greatly upregulated in lesions, whereas a significant decrease in expression of Tie2 and ligands occur under anti-psoriatic treatment (Kuroda et al. J. Invest. Dermatol 2001, 116, 713). Direct association of pathogenesis of disease with Tie2 expression has been demonstrated recently in transgenic mice overexpressing Tie2 (Voskas et al. Am. J. Pathol. 2005, 166, 843). In these mice overexpression of Tie2 causes a psoriasis-like phenotype (such as epidermal thickening, rete ridges and lymphocyte infiltration).These skin abnormalities are resolved completely upon suppression of transgene expression, thereby illustrating a complete dependence on Tie2 signalling for disease maintenance and progression.

Tie2 expression was investigated in human breast cancer specimens and Tie2 expression was found in the vascular endothelium both in normal breast tissue as well as in tumour tissue. The proportion of Tie2-positive microvessels was increased in tumours as compared to normal breast tissue (Peters et al. Br. J. Canc. 1998, 77, 51). However, significant heterogeneity in endothelial Tie2 expression was observed in clinical specimen from a variety of human cancers (Fathers et al. Am. J. Path. 2005, 167, 1753). In contrast, Tie2 and angiopoietins were found to be highly expressed in the cytoplasm of human colorectal adenocarcinoma cells indicating at the potential presence of an autocrine/paracrine growth loop in certain cancers (Nakayama et al. World J. Gastroenterol. 2005, 11, 964). A similar autocrine/paracrine Ang1-Ang2-Tie2 loop was postulated for certain human gastric cancer cell lines (Wang et al. Biochem. Biophys. Res. Comm. 2005, 337, 386).

The relevance of the Ang1-Tie2 signalling axis was challenged with various biochemical techniques. Inhibition of Ang1 expression by an antisense RNA approach resulted in decreased xenograft tumour growth (Shim et al. Int. J. Canc. 2001, 94, 6 ; Shim et al. Exp. Cell Research 2002, 279, 299). However, other studies report that experimental overexpression of Ang1 in tumour models leads to decreased tumour growth (Hayes et al. Br. J. Canc. 2000, 83, 1154; Hawighorst et al. Am. J. Pathol. 2002, 160, 1381; Stoeltzing et al. Cancer Res. 2003, 63, 3370). The latter results can be rationalized by the ligand's ability to stabilize the endothelial lining of vessels rendering vessels less sensitive for angiogenic stimuli. Interference with the dynamics of Ang1-Tie2 signalling either by over-stimulation or by stimulus deprivation seemingly leads to similar phenotypes.

The pharmacological relevance of inhibiting Tie2 signalling was tested applying various non-small molecule approaches. A peptidic inhibitor of Ang1/2 binding to Tie2 was shown to inhibit Ang1-induced HUVEC migration and angiogenesis induction in an in vivo model (Tournaire et al. EMBO Rep. 2005, 5, 1). Corneal angiogenesis induced by tumour cell conditioned medium was inhibited by a recombinant soluble Tie2 receptor (sTie2) despite the presence of VEGF (Lin et al. J. Clin. Invest. 1997, 100, 2072; see also Singh et al. Biochem. Biophys. Res. Comm. 2005, 332, 194). Gene therapy by adenoviral vector delivered sTie2 was capable of reducing tumour growth rates of a murine mammary carcinoma and a murine melanoma and resulted in reduction of metastasis formation (Lin et al. Proc. Natl. Acad. Sci. USA 1998, 95, 8829). Similar effects were observed with related sTie2 constructs (Siemeister et al. Cancer Res. 1999, 59, 3185) and a Tek-Fc construct (Fathers et al. Am. J. Path. 2005, 167, 1753).

Adenovirus-delivered anti-Tie2 intrabodies were shown to inhibit growth of a human Kaposi's sarcoma and a human colon carcinoma upon peritumoural administration (Popkov et al. Cancer Res. 2005, 65, 972). Histopathological analysis revealed a marked decrease in vessel density in treated vs. control tumours. Phenotypic simultaneous knockout of KDR and Tie2 by an adenovirus delivered intradiabody resulted in significantly higher growth inhibition of a human melanoma xenograft model than KDR knockout alone (Jendreyko et al. Proc. Natl. Acad. Sci. USA 2005, 102, 8293). Similarly, the bispecific Tie2-KDR intradiabody was more active in an *in vitro* EC tube formation inhibition assay than the two monospecific intrabodies alone (Jendreyko et al. J. Biol. Chem. 2003, 278, 47812). Systematic treatment of tumour-bearing mice with Ang2-blocking antibodies and peptide-Fc fusion proteins led to tumour stasis and elimination of tumour burden in a subset of animals (Oliner et al. Cancer Cell 2004, 6, 507). For a recent report on an immunization approach, see Luo et al. Clin. Cancer Res. 2006, 12, 1813.

However, from the above studies using biochemical techniques to interfere with Tie2 signalling it is not clear, whether similar phenotypes will be observed with small molecule inhibitors of the Tie2 kinase activity. Small molecule inhibitors of kinases by definition block only those cellular effects which are mediated by the receptor's kinase activity and not those which might involve the kinase only as a co-receptor or scaffolding component in multi-enzyme complexes. So far, only a single study using a small molecule Tie2 inhibitor has been published (Scharpfenecker et al. J. Cell Sci. 2005, 118, 771). It remains to be shown that small molecule inhibitors of the Tie2 kinase will be as efficacious in inhibiting angiogenesis as e.g. ligand antibodies, soluble decoy receptors or receptor intrabodies. As discussed above, in certain settings inhibition of Tie2 signalling alone might not be sufficient to induce an adequate antiangiogenic effect. Simultaneous inhibition of several angiogenesis relevant signalling pathways could overcome such inadequacies. In conclusion, there is a great need for novel chemotypes for small mocule inhibitors of the Tie2 kinase. Fine tuning of additive anti-angiogenic activities as well as pharmacokinetic parameters such as e.g. solubility, membrane permeability, tissue distribution and metabolism will finally allow for chosing compounds of accurate profiles for various diseases caused by or associated with dysregulated vascular growth.

To date, a small number of therapeutic agents with antiangiogenic activity have been approved for cancer treatment. Avastin (Bevacizumab), a VEGF neutralizing antibody, blocks KDR and VEGFR1 signalling and has been approved for first-line treatment of metastatic colorectal cancer. The small molecule multi-targeted kinase inhibitor Nexavar (Sorafenib) inhibits *inter alia* members of the VEGFR family and has been approved for the treatment of advanced renal cell carcinoma. Sutent (Sunitinib), another multi-targeted kinase inhibitor with activity vs. VEGFR family members, has been approved by the FDA for treatment of patients with gastrointestinal stromal tumours (GIST) or advanced kidney tumours. Several other small molecule inhibitors of angiogenesis-relevant targets are in clinical and preclinical development.

AMG-386, an angiopoietin-targeting recombinant Fc fusion protein, is in phase I clinical development in patients with advanced solid tumours. Several multi-targeted small molecule inhibitors with activity against Tie2 are (or have been) in preclinical evaluation for cancer therapy, including ABT-869, GW697465A and A-422885.88 (BSF466895). The first and most recent compound, however, was reported to possess higher inhibitory activity against other kinase targets including non-angiogenesis kinases and oncogenic kinases. This agent is therefore not considered to be a purely antiangiogenic agent and its applicability to non-cancer diseases remains to be shown.

Pyrimidines and their derivatives have been frequently described as therapeutic agents for diverse diseases. Various recently published patent applications describe their use as inhibitors of protein kinases, for example in WO2001064654 and WO 2002096888 for use as CDK inhibitors, in WO 2003032997 for use as CDK and Aurora A kinase inhibitors, in WO 2003063794 for use as Syk kinase inhibitors, in WO 2003078404 for use as ZAP-70 and/or Syk or FAK kinase inhibitors, in WO 2004074244 for use as PLK inhibitors, in WO 2005026158 as ZAP-70 and/or Syk kinase inhibitors, and in WO 2005026130 as Alk inhibitors.

More specifically, certain 2,4-diaminosubstituted pyrimidine derivatives have been disclosed as inhibitors of protein kinases involved in angiogenesis, such as VEGFR-2 (KDR) and/or Tie2 kinase, for example benzimidazole substituted 2,4-diaminopyrimidines (WO 2003074515) or bis-2,4-anilino-pyrimidines (WO 2003066601).

Incorporation of pyrimidines into a bicyclic structure has been reported to provide compounds with Tie2/VEGFR-2 dual inhibitory activity (WO 2003022582). Very recently, pyrimidine derivatives in which the pyrimidine constitutes a part of a macrocyclic ring system have been reported to be inhibitors of CDKs and/or VEGFRs (WO 2004026881), or of CDK2 and/or CDK5, respectively (WO 2004078682).

A problem in using substituted 2,4-diaminopyrimidines for the treatment of diseases associated with dysregulated vascular growth is their often poor selectivity for angiogenesis relevant kinases, which are primarily expressed on endothelial cells (EC), over kinases expressed in proliferating non-endothelial cells (e.g. cell cycle modulating kinases). By affecting not only the endothelium of aberrantly growing vasculature but also proliferating non endothelial cells, prolonged treatment with such compounds may cause side effects which would limit their pharmaceutical applicability. The patent applications cited above render diaminopyrimidines to be well known cell cycle kinase inhibitors, more particularly potent CDK and/or Aurora kinase inhibitors. However, it would be highly advantageous to have compounds at one's disposal which target one or more angiogenesis-relevant kinases while possessing modest or, even more advantageously, negligible activity against cell cycle modulating kinases such as CDK2 and/or Aurora kinases. Such a pharmacological profile is highly desirable for treating diseases of dysregulated vascular growth or diseases which are accompanied with dysregulated vascular growth, in particular solid tumours and metastases thereof, and is even more desirable for treating non-oncological diseases of dysregulated vascular growth or non-oncological diseases which are accompanied with dysregulated vascular growth, such as retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel, and diseases such as coronary and peripheral artery disease.

Therefore the aim of the present invention is to provide compounds which are useful for the treatment of diseases resulting from or associated with dysregulated vascular growth. Selective inhibitors of one or multiple kinases, which are expressed on endothelial cells and play a role in the angiogenic sprouting, remodelling and/or maturation of new blood vessels, shall be provided. More particularly, potent inhibitors of Tie2 kinase shall be provided. Furthermore the prior art problems shall be overcome by providing compounds which do not inhibit CDK2 and Aurora C at compound concentrations of relevance for their Tie2 inhibitory activity.

The solution to the above-mentioned novel technical problem is achieved by providing compounds derived, in accordance with the present invention, from a class of sulfonamido-macrocycles and salts thereof, methods of preparing sulfonamido-macrocycles, a pharmaceutical composition containing said sulfonamido-macrocycles, uses of said sulfonamido-macrocycles and a method for treating diseases with said sulfonamido-macrocycles, all in accordance with the description, as defined in the claims of the present application.

The present invention thus relates to compounds of general formula (I) : 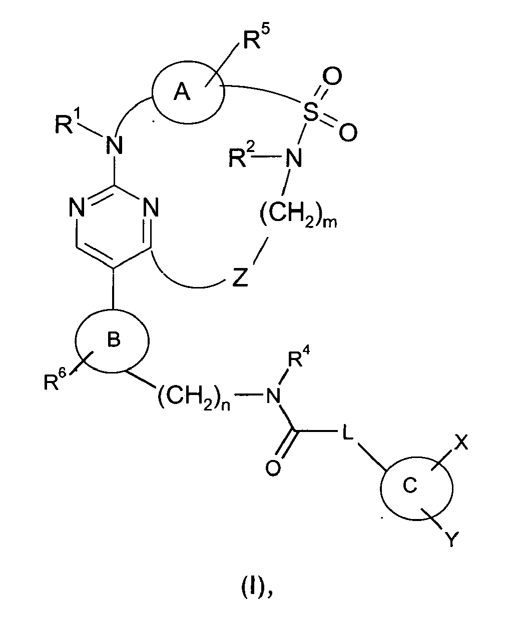 wherein
- A: is phenylene or C₆-heteroarylene ;
- B, C: are, independently from each other, arylene or heteroarylene ;
- Z: is selected from the group comprising, preferably consisting of, O, S, NR³ and C_{HR}³ ;
- R¹, R², R³ and R⁴: independently from each other are selected from the group comprising, preferably consisting of, hydrogen and C₁-C₆-alkyl, wherein C₁-C₆-alkyl is unsubstituted or substituted one or more times with -OR⁷ or -NR⁷R⁸ ;
- R⁵: is selected from the group comprising, preferably consisting of, hydrogen, halogen, nitro, cyano, C₁-C₆-alkyl, -(CH₂)ₚOR^{7a}, - (CH₂)ₚNR^{7a}R^{8a}, -(CH₂)ₚC(O)R^{7a}, -(CH₂)ₚNHC(O)R^{7a}, - (CH₂)ₚNHC(O)NR^{7a}R^{8a}, -(CH₂)ₚNHS(O)₂R^{7a}, and -(CH₂)ₚC(O)NR^{7a}R^{8a} ;
- R⁶: is selected from the group comprising, preferably consisting of, hydrogen, halogen, nitro, cyano, C₁-C₆-alkyl, -OR^{7b}, -NR^{7b}R^{8b}, C₁-C₆-haloalkyl, C₁-C₆-haloalkoxy, C₁-C₆-alkylthio and C₁-C₆-alkylcarbonyl ;
- R⁷, R^{7a}, R^{7b}, R^{7c}, R^{7d}, R^{7e}, R^{7f} , R⁸, R^{8a}, R^{8b} , R^{8c}, R^{8d}, R^{8e}, R^{8f}: independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7c}, -NR^{7c} R^{8c}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a} ; or
- R⁷ and R⁸, R^{7a} and R^{8a}, R^{7b} and R^{8b},:
- R^{7c} and R^{8c} , R^{7e} and R^{8e}, and R^{7f} and R^{8f}: independently from each other, together with the nitrogen atom to which they are attached in groups -NR⁷R⁸ -NR^{7a}R^{8a}, -NR^{7b}R^{8b}, -NR^{7c}R^{8c}, -NR^{7e}R^{8e}, and -NR^{7f}R^{8f} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a - C(O)-, -S(O)- , and -S(O)₂- group, and can optionally contain one or more double bonds ;
- R⁹, R^{9a},: independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
- R⁹ and R^{9a},: together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- L: is selected from the group comprising, preferably consisting of C₁-C₆-alkylene or C₃-C₁₀-cycloalkylene ;
- X: is selected from the group comprising, preferably consisting of, halogen, nitro, cyano, -(CH₂)ᵣOR^{7e}, -(CH₂)ᵣNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7e}, -(CH₂)ᵣC(O)NR^{7e}R^{8e} and - (CH₂)ᵣS(O)₂NR^{7e}R^{8e} ;
- Y: is selected from the group comprising, preferably consisting of, hydrogen, halogen, nitro, cyano, -(CH₂)ₛOR^{7f}, -(CH₂)ₛNR^{7f}R^{8f}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7f}, -(CH₂)ₛC(O)NR^{7f}R^{8f} and - (CH₂)ₛS(O)₂NR^{7f}R^{8f} ;
- m: represents an integer of 3, 4, 5, or 6 ;
- n, p, q, q', r, s: independently from each other represent an integer of 0, 1, or 2 .

In a preferred embodiment, the present invention relates to compounds of general formula (I) supra, wherein :
- A: is meta-phenylene ;
- B, C: are, independently from each other, arylene or heteroarylene ;
- Z: is NR³;
- R¹, R², R³, and R⁵: are hydrogen ;
- R⁴: is hydrogen or C₁-C₆-alkyl ;
- R⁶: is selected from the group comprising, preferably consisting of, hydrogen, halogen, nitro, cyano, C₁-C₆-alkyl, -OR^{7b}, -NR^{7b}R^{8b} , C₁-C₆-haloalkyl, C₁-C₆-haloalkoxy, C₁-C₆-alkylthio and C₁-C₆-alkylcarbonyl ;
- R^{7b}, R^{7c}, R^{7d}, R^{7e}, R^{7f} , R^{8b} R^{8c} R^{8d} , R^{8e}, R^{8f}: independently from each other, represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7c}, -NR^{7c}R^{8c} , C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a} ; or
- R^{7b} and R^{8b}, R^{7c} and R^{8c}, R^{7e} and R^{8e}, and R^{7f} and R^{8f}: independently from each other, together with the nitrogen atom to which they are attached in groups -NR^{7b}R^{8b}, -NR^{7c}R^{8c}, -NR^{7e}R^{8e}, and - NR^{7f}R^{8f} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- R⁹ R^{9a},: independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
- R⁹ and R^{9a},: together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- L: is selected from the group comprising, preferably consisting of C₁-C₆-alkylene or C₃-C₁₀-cycloatkylene ;
- X: is selected from the group comprising, preferably consisting of, halogen, nitro, cyano, -(CH₂)ᵣOR^{7e}, -(CH₂)ᵣNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7e}, -(CH₂)ᵣC(O)NR^{7e}R^{8e} and - (CH₂)ᵣS(O)₂NR^{7e}R^{8e} ;
- Y: is selected from the group comprising, preferably consisting of, hydrogen, halogen, nitro, cyano, -(CH₂)ₛOR^{7f}, -(CH₂)ₛNR^{7f}R^{8f}, C₁-C₆-alkyl, C₃-C₁₀-cycloalkyl, C₃-C₁₀-heterocycloatkyl, C₁-C₆-haloalkyl, C₁-C₆-haloalkoxy, C₁-C₆-alkylthio, -(CH₂)ₛC(O)R^{7f}, -(CH₂)ₛC(O)NR^{7f}R^{8f} and - (CH₂)ₛS(O)₂NR^{7f}R^{8f};
- m: represents an integer of 3 ; and
- n, q, q', r,: s independently from each other represent an integer of 0, 1, or 2 .

In a more preferred embodiment, the present invention relates to compounds of general formula (I), supra, in which :
- A: is meta-phenylene ;
- B: is phenylene ;
- C: is arylene or heteroarylene ;
- Z: is NR³ ;
- R¹, R², R³ and R⁵: are hydrogen ;
- R⁴: is hydrogen or C₁-C₆-alkyl ;
- R⁶: is selected from the group comprising, preferably consisting of, hydrogen, halogen, methyl, -OH ;
- R^{7c}, R^{7d}, R^{7e}, R^{7f}, R^{8c}, R^{8d}, R^{8e}, R^{8f}: independently from each other, represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)q-aryl and -(CH₂)_{q}-heteroaryl, wherein said residues are unsubstituted or substituted one or more times independently from each other with halogen, nitro, cyano, C₁-C₆-alkyl, -OR^{7c}, -NR^{7c}R^{8c}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
- R^{7c} and R^{8c}, R^{7e} and R^{8e}, and R^{7f} and R^{8f}: independently from each other, together with the nitrogen atom to which they are attached in groups -NR^{7c}R^{8c}, -NR^{7c}R^{8c}, and -NR^{7f}R^{8f} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- R⁹, R^{9a},: independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
- R⁹ and R^{9a},: together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂. group, and can optionally contain one or more double bonds ;
- L: is selected from the group comprising, preferably consisting of C₁-C₆-alkylene or C₃-C₁₀-cycloalkylene ;
- X: is selected from the group comprising, preferably consisting of, halogen, nitro, cyano, -(CH₂)ᵣOR^{7e}, -(CH₂)ᵣNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7e}, -(CH₂)ᵣC(O)NR^{7e}R^{8e} and - (CH₂)ᵣS(O)₂NR^{7e}R^{8e} ;
- Y: is selected from the group comprising, preferably consisting of, hydrogen, halogen, nitro, cyano, -(CH₂)ₛOR^{7f}, -(CH₂)ₛNR^{7f}R^{8f}, C₁-C₆-alkyl, C₃-C₁₀-cycLoaLkyL, C₃-C₁₀-heterocycloalkyl, C₁-C₆-haloalkyl, C₁-C₆-haloalkoxy, C₁-C₆-alkylthio, -(CH₂)ₛC(O)R^{7f}, -(CH₂)ₛC(O)NR^{7f}R^{8f} and - (CH₂)ₛS(O)₂NR^{7f}R^{8f} ;
- m: represents an integer of 3 ; and
- n, q, q', r,: s independently from each other represent an integer of 0, 1, or 2 .

In a more particularly preferred embodiment, the present invention relates to compounds of general formula (I), supra, in which :
- A: is meta-phenylene ;
- B: is para-phenylene ;
- C: is phenylene ;
- Z: is NR³ ;
- R¹, R², R³, R⁴, and R⁵: are hydrogen ;
- R⁶: is selected from the group comprising, preferably consisting of, hydrogen, fluoro ;
- R^{7c}, R^{7d}, R^{7e}, R^{7f} R^{8c} R^{8e}: independently from each other, represent hydrogen, or are selected from the group comprising, preferably consisting of, C₁-C₆-alkyl, C₃-C₆-cycloalkyl, wherein said residues are unsubstituted or substituted one or more times independently from each other with -OR^{7c} or - NR^{7c}R^{8c} ; or
- R^{7c} and R^{8c}, and R^{7e} and R^{8e}: independently from each other, together with the nitrogen atom to which they are attached in groups -NR^{7c}R^{8c}, -NR^{7e}R^{8e}, and form a 3 to 6 membered heterocyctoalkyl ring, wherein the carbon backbone of this heterocycloalkyl ring can optionally be interrupted one or more times, the same way or differently, by a member of the group comprising, preferably consisting of, NH, NR^{7d} or oxygen ;
- L: is selected from the group comprising, preferably consisting of C₁-C₃-alkylene or C₃-C₆-cycloalkylene ;
- X: is selected from the group comprising, preferably consisting of, halogen, -(CH₂)ᵣOR^{7e}, -(CH₂)ᵣNR^{7e}R^{8e}, C₁-C₆-alkyl, C₁-C₆-haloalkyl, C₁-C₆-haloalkoxy ;
- Y: is selected from the group comprising, preferably consisting of, hydrogen, halogen, -(CH₂)ₛOR^{7f}, C₁-C₆-alkyl, C₁-C₆-haloalkyl ;
- m: represents an integer of 3 ;
- n and r: independently from each other represent an integer of 0 or 1 ; and
- s: represents an integer of 0.

In an even more particularly preferred embodiment, the present invention relates to compounds of general formula (I), supra, in which :
- A: is meta-phenylene ;
- B: is para-phenylene ;
- C: is phenylene ;
- Z: is NR³;
- R¹, R², R³, R⁴, R⁵, and R⁶: is hydrogen ;
- R^{7c}, R^{7d}, R^{7e}, R^{7f}, R^{8c}, R^{8e}: independently from each other, represent hydrogen, or C₁-C₆-alkyl, wherein said C₁-C₆-alkyl residues are unsubstituted or substituted one or more times independently from each other with -OR^{7c} or -NR^{7c}R^{8c}; or
- R^{7c} and R^{8c}, and R^{7e} and R^{8e}: independently from each other, together with the nitrogen atom to which they are attached in groups -NR^{7c}R^{8c}, -NR^{7e}R^{8e}, and form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d} or oxygen ;
- L: is selected from the group comprising, preferably consisting of C₁-C₃-alkylene or C₃-cycloalkylene ;
- X: is selected from the group comprising, preferably consisting of, halogen, -(CH₂)ᵣOR^{7e}, -(CH₂)ᵣNR^{7e}R^{8e}, C₁-C₆-alkyl, C₁-C₆-haloalkyl, C₁-C₆-hatoalkoxy ;
- Y: is selected from the group comprising, preferably consisting of, hydrogen, halogen, -(CH₂)ₛOR^{7f}, C₁-C₆-alkyl, C₁-C₆-haloalkyl ;
- m: represents an integer of 3 ;
- n: represents an integer of 0 ;
- r: represents an integer of 0 or 1 ; and
- s: represents an integer of 0.

The terms as mentioned herein and in the claims have preferably the following meanings :

The term "alkyl", as used in the context of the term "alkyl" or "alkylcarbonyl", for example, is to be understood as preferably meaning branched and unbranched alkyl, meaning e.g. methyl, ethyl, n-propyl, iso-propyl, n-butyl, iso-butyl, *tert-*butyl, sec-butyl, pentyl, iso-pentyl, hexyl, heptyl, octyl, nonyl and decyl and the isomers thereof.

The term "haloalkyl" is to be understood as preferably meaning branched and unbranched alkyl, as defined *supra,* in which one or more of the hydrogen substituents is replaced in the same way or differently with halogen. Particularly preferably, said haloalkyl is, e.g. chloromethyl, fluoropropyl, fluoromethyl, difluoromethyl, trichloromethyl, 2,2,2-trifluoroethyl, pentafluoroethyl, bromobutyl, trifluoromethyl, iodoethyl, and isomers thereof.

The term "alkoxy" is to be understood as preferably meaning branched and unbranched alkoxy, meaning e.g. methoxy, ethoxy, propyloxy, iso-propyloxy, butyloxy, iso-butyloxy, tert-butyloxy, sec-butyloxy, pentyloxy, iso-pentyloxy, hexyloxy, heptyloxy, octyloxy, nonyloxy, decyloxy, undecyloxy and dodecyloxy and the isomers thereof.

The term "alkylthio" is to be understood as preferably meaning branched and unbranched alkylthio, meaning e.g. methylthio, ethylthio, propylthio, *iso-*propylthio, butylthio, *iso*-butylthio, *tert*-butylthio, *sec*-butylthio, pentylthio, *iso-*pentylthio, hexylthio, heptylthio, octylthio, nonylthio, decylthio, undecylthio and dodecylthio and the isomers thereof.

The term "haloalkoxy" is to be understood as preferably meaning branched and unbranched alkoxy, as defined *supra,* in which one or more of the hydrogen substituents is replaced in the same way or differently with halogen, e.g. chloromethoxy, fluoromethoxy, pentafluoroethoxy, fluoropropyloxy, difluoromethyloxy, trichloromethoxy, 2,2,2-trifluoroethoxy, bromobutyloxy, trifluoromethoxy, iodoethoxy, and isomers thereof.

The term "cycloalkyl" is to be understood as preferably meaning a C₃-C₁₀ cycloalkyl group, more particularly a saturated cycloalkyl group of the indicated ring size, meaning e.g. a cyclopropyl, cyclobutyl, cyclopentyl, cyclohexyl, cycloheptyl, cyclooctyl, cyclononyl, or cyclodecyl group ; and also as meaning an unsaturated cycloalkyl group containing one or more double bonds in the C-backbone, e.g. a C₃-C₁₀ cycloalkenyl group, such as, for example, a cyclopropenyl, cyclobutenyl, cyclopentenyl, cyclohexenyl, cycloheptenyl, cyclooctenyl, cyclononenyl, or cyclodecenyl group, wherein the linkage of said cyclolalkyl group to the rest of the molecule can be provided to the double or single bond.

The term "heterocycloalkyl" is to be understood as preferably meaning a C₃-C₁₀ cycloalkyl group, as defined *supra,* featuring the indicated number of ring atoms, wherein one or more ring atoms are heteroatoms such as NH, NR^{8d}, oxygen or sulfur, or carbonyl groups, or, -otherwise stated - in a Cₙ-cycloalkyl group one or more carbon atoms are replaced by these heteroatoms to give such Cₙ cycloheteroalkyl group. Thus such group refers *e.g.* to a three-membered heterocycloalkyl, expressed as -C₃-heterocycloalkyl such as oxyranyl. Other examples of heterocycloalkyls are oxetanyl (C₄), aziridinyl (C₃), azetidinyl (C₄), tetrahydrofuranyl (C₅), pyrrolidinyl (C₅), morpholinyl (C₆), dithianyl (C₆), thiomorpholinyl (C₆), piperazinyl (C₆), trithianyl (C₆) and chinuclidinyl (C₈).

The term "halogen" or "Hal" is to be understood as preferably meaning fluorine, chlorine, bromine, or iodine.

The term "alkenyl" is to be understood as preferably meaning branched and unbranched alkenyl, e.g. a vinyl, propen-1-yl, propen-2-yl, but-1-en-1-yl, but-1-en-2-yl, but-2-en-1-yt, but-2-en-2-yl, but-1-en-3-yl, 2-methyl-prop-2-en-1-yl, or 2-methyl-prop-1-en-1-yl group.

The term "alkynyl" is to be understood as preferably meaning branched and unbranched alkynyl, *e.g.* an ethynyl, prop-1-yn-1-yl, but-1-yn-1-yl, but-2-yn-1-yl,or but-3-yn-1-yl group.

As used herein, the term "aryl" is defined in each case as having 3-12 carbon atoms, preferably 6-12 carbon atoms, such as, for example, cyclopropenyl, cyclopentadienyl, phenyl, tropyl, cyclooctadienyl, indenyl, naphthyl, azulenyl, biphenyl, fluorenyl, anthracenyl etc, phenyl being preferred.

As used herein, the term "heteroaryl" is understood as meaning an aromatic ring system which comprises 3-16 ring atoms, preferably 5 or 6 or 9 or 10 atoms, and which contains at least one heteroatom which may be identical or different, said heteroatom being such as oxygen, nitrogen or sulfur, and can be monocyclic, bicyclic, or tricyclic, and in addition in each case can be benzocondensed. Preferably, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl etc., and benzo derivatives thereof, such as, e.g., benzofuranyl, benzothienyl, benzoxazolyl, benzimidazolyl, benzotriazolyl, indazolyl, indolyl, isoindolyl, etc.; or pyridyl, pyridazinyl, pyrimidinyl, pyrazinyl, triazinyl, etc., and benzo derivatives thereof, such as, for example, quinolinyl, isoquinolinyl, *etc.;* or azocinyl, indolizinyl, purinyl, *etc.,* and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, etc.

The term "alkylene", as used herein in the context of the compounds of general formula (I) is to be understood as meaning an optionally substituted alkyl chain or "tether", having 1, 2, 3, 4, 5, or 6 carbon atoms, *i.e.* an optionally substituted - CH₂- ("methylene" or "single membered tether" or e.g. -C(Me)₂-), -CH₂-CH₂-("ethylene", "dimethylene", or "two-membered tether"), -CH₂-CH₂-CH₂-("propylene", "trimethylene", or "three-membered tether"), -CH₂-CH₂-CH₂-CH₂-("butylene", "tetramethylene", or "four-membered tether"), -CH₂-CH₂-CH₂-CH₂-CH₂- ("pentylene", "pentamethylene" or "five-membered ether"), or -CH₂-CH₂-CH₂-CH₂-CH₂-CH₂- ("hexylene", "hexamethylene", or six-membered tether") group. Preferably, said alkylene tether is 1, 2, 3, 4, or 5 carbon atoms, more preferably 1 or 2 carbon atoms.

The term "cycloalkylene", as used herein in the context of the compounds of general formula (I) is to be understood as meaning an optionally substituted cycloalkyl ring, having 3, 4, 5, 6, 7, 8, 9 or 10, preferably 3, 4, 5, or 6, carbon atoms, *i.e.* an optionally substituted cyclopropyl, cyclobutyl, cyclopentyl, cyclohexyl, cycloheptyl, cyclooctyl, cyclononyl, or cyclodecyl ring, preferably a cyclopropyl, cyclobutyl, cyclopentyl or cyclohexyl ring.

The term "heterocycloalkylene", as used herein in the context of the compounds of general formula (I) is to be understood as meaning a cycloalkylene ring, as defined *supra,* but which contains at least one heteroatom which may be identical or different, said heteroatom being such as oxygen, nitrogen or sulfur.

The term "phenylene", as used herein in the context of the compounds of general formula (I) which include the group A, is to be understood as meaning an optionally substituted all-carbon monocyclic six-membered arylene aromatic system or "tether". When the term "phenylene" is used it is to be understood that the linking residues can be arranged to each other in ortho-, para- or meta-position.

The term "arylene", as used herein in the context of the compounds of general formula (I) which include the groups B and C, is to be understood as meaning an optionally substituted monocyclic or polycyclic arylene aromatic system e.g. arylene, naphthylene and biarylene, preferably an optionally substituted phenyl ring or "tether", having 6 or 10 carbon atoms. More preferably, said arylene tether is a ring having 6 carbon atoms. When the term "arylene" is used it is to be understood that the linking residues can be arranged to each other in ortho-, para-or meta-position, e.g.an optionally substituted moiety of structure : 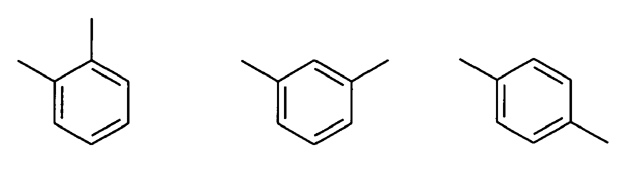 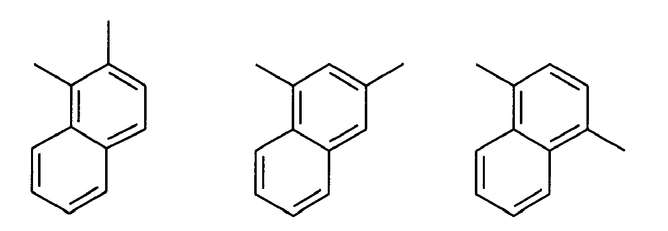 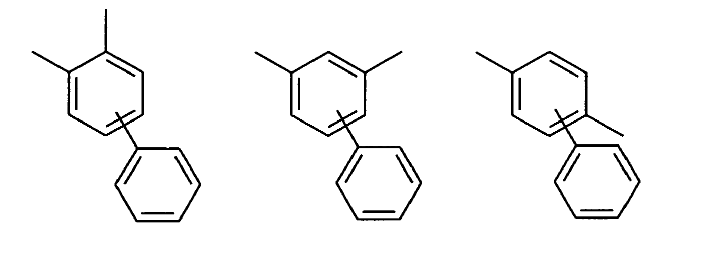 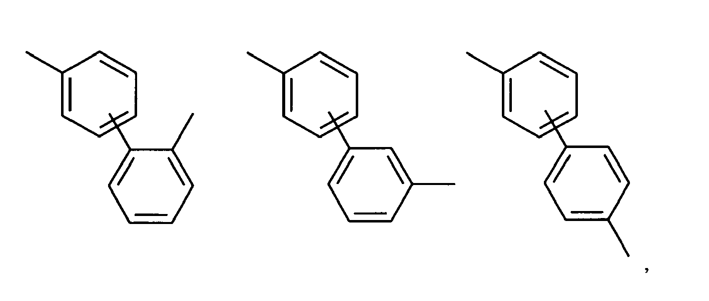 in which linking positions on the rings are shown as non-attached bonds.

The term "heteroarylene", as used herein in the context of the compounds of general formula (I) which include the groups A, B and C, is to be understood as meaning an optionally substituted monocyclic or polycyclic heteroarylene aromatic system, e.g. heteroarylene, benzoheteroarylene, preferably an optionally substituted 5-membered heterocycle, such as, for example, furan, pyrrole, thiazole, oxazole, isoxazole, or thiophene or "tether", or a 6-membered heterocycle, such as, for example, pyridine, pyrimidine, pyrazine, pyridazine. More preferably, said heteroarylene tether is a ring having 6 carbon atoms, e.g. an optionally substituted structure as shown *supra* for the arylene moieties, but which contains at least one heteroatom which may be identical or different, said heteroatom being such as oxygen, nitrogen or sulfur. When the term "heteroarylene" is used it is to be understood that the linking residues can be arranged to each other in ortho-, para- or meta-position.

As used herein, the term "C₁-C₆", as used throughout this text, e.g. in the context of the definition of "C₁-C₆-alkyl", or "C₁-C₆-alkoxy", is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, e.g. C₁-C₆, C₂-C₅, C₃-C₄, C₁-C₂ , C₁-C₃ , C₁-C₄, C₁-C₅ C₁-C_{6;} preferably C₁-C₂, C₁-C_{3,} C₁-C_{4,} C₁-C_{5,} C₁-C_{6;} more preferably C₁-C₄.

Similarly, as used herein, the term "C₂-C₆", as used throughout this text, e.g. in the context of the definitions of "C₂-C₆-alkenyl" and "C₂-C₆-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "C₂-C₆" is to be interpreted as any sub-range comprised therein, e.g. C₂-C₆, C₃-C₅ , C₃-C₄, C₂-C₃, C₂-C₄, C₂-C₅; preferably C₂-C₃ .

As used herein, the term "C₃-C₁₀", as used throughout this text, e.g. in the context of the definitions of "C₃-C₁₀-cycloalkyl", "C₃-C₁₀-heterocycloalkyL", or "C₃-C₁₀-cycloalkylene" is to be understood as meaning a cycloalkyl or heterocycloalkyl group or a cycloalkylene tether having a finite number of carbon atoms of 3 to 10, *i.e.* 3, 4, 5, 6, 7, 8, 9 or 10 carbon atoms, preferably 3, 4, 5 or 6 carbon atoms. It is to be understood further that said term "C₃-C₁₀" is to be interpreted as any sub-range comprised therein, e.g. C₃-C₁₀, C₄-C₉, C₅-C₈, C₆-C₇; preferably C₃-C_{6.}

As used herein, the term "C₃-C₆", as used throughout this text, e.g. in the context of the definitions of "C₃-C₆-cycloalkyl", "C₃-C₆-heterocycloalkyl", or "C₃-C₆-cycloalkylene" is to be understood as meaning a cycloalkyl group or a heterocycloalkyl group or a cycloalkyl tether having a finite number of carbon atoms of 3 to 6, *i. e.* 3, 4, 5, or 6 carbon atoms. It is to be understood further that said term "C₃-C₆" is to be interpreted as any sub-range comprised therein, e.g. C₃-C₄, C₄-C₆ , C₅-C₆ .

Further, as used herein, the term "C₃-C₈", as used throughout this text e.g. in the context of the definitions of "C₃-C₈-cycloalkyl", is to be understood as meaning a cycloalkyl group having a finite number of carbon atoms of 3 to 8, i.e. 3, 4, 5, 6, 7 or 8 carbon atoms. It is to be understood further that said term "C₃-C₈" is to be interpreted as any sub-range comprised therein, e.g. C₃-C₈, C₄-C₇, C₅-C₆, C₃-C₄, C₃-C₅, C₃-C₆, C₃-C₇.

As used herein, the term "C₆-C₁₁", as used throughout this text, e.g. in the context of the definitions of "C₆-C₁₁-aryl", is to be understood as meaning an aryl group having a finite number of carbon atoms of 5 to 11, *i.e.* 5, 6, 7, 8, 9, 10 or 11 carbon atoms, preferably 5, 6, or 10 carbon atoms. It is to be understood further that said term "C₆-C₁₁" is to be interpreted as any sub-range comprised therein, e.g. C₅-C₁₀, C₆-C₉, C₇-C₈; preferably C₅-C₆.

As used herein, the term "C₅-C₁₀", as used throughout this text, e.g. in the context of the definitions of "C₅-C₁₀-heteroaryl", is to be understood as meaning a heteroaryl group having a finite number of carbon atoms of 5 to 10, in addition to the one or more heteroatoms present in the ring *i.e.* 5, 6, 7, 8, 9, or 10 carbon atoms, preferably 5, 6, or 10 carbon atoms. It is to be understood further that said term "C₅-C₁₀" is to be interpreted as any sub-range comprised therein, *e.g.* C₆-C₉, C₇-C₈, C₇-C₈ ; preferably C₅-C₆.

As used herein, the term "C₁-C₃", as used throughout this text, e.g. in the context of the definitions of "C₁-C₃-alkylene", is to be understood as meaning an alkylene group as defined *supra* having a finite number of carbon atoms of 1 to 3, *i.e.* 1, 2, or 3. It is to be understood further that said term "C₁-C₃" is to be interpreted as any sub-range comprised therein, e.g. C₁-C₂, or C₂-C₃.

The term "isomers" is to be understood as meaning chemical compounds with the same number and types of atoms as another chemical species. There are two main classes of isomers, constitutional isomers and stereoisomers.

The term "constitutional isomers" is to be understood as meaning chemical compounds with the same number and types of atoms, but they are connected in differing sequences. There are functional isomers, structural isomers, tautomers or valence isomers.

In stereoisomers, the atoms are connected sequentially in the same way, such that condensed formulae for two isomeric molecules are identical. The isomers differ, however, in the way the atoms are arranged in space. There are two major subclasses of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers. Diastereomers which still have a different constitution, are another sub-class of diastereomers, the best known of which are simple *cis - trans* isomers.

In order to limit different types of isomers from each other reference is made to IUPAC Rules Section E (*Pure Appl Chem* 45, 11-30, 1976).

The compound according to Fomula (I) can exist in free form or in a salt form. A suitably pharmaceutically acceptable salt of the sulfonamido-macrocycles of the present invention may be, for example, an acid-addition salt of a sulfonamido-macrocycle of the invention which is sufficiently basic, for example, an acid-addition salt with, for example, an inorganic or organic acid, for example hydrochloric, hydrobromic, sulfuric, phosphoric, trifluoroacetic, paratoluenesulfonic, methylsulfonic, citric, tartaric, succinic or maleic acid. In addition, another suitably pharmaceutically acceptable salt of a sulfonamido-macrocycle of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methylglucamine, dimethyl-glucamine, ethyl-glucamine, lysine, 1,6-hexadiamine, ethanolamine, glucosamine, sarcosine, serinol, tris-hydroxy-methyl-aminomethane, aminopropandiol, sovak-base, 1-amino-2,3,4-butantriol.

The compound according to Formula (I) can exist as N-oxides which are defined in that at least one nitrogen of the compounds of the general Formula (I) may be oxidized.

The compound according to Formula (I) can exist as solvates, in particular as hydrate, wherein the compound according to Formula (I) may contain polar solvents, in particular water, as structural element of the crystal lattice of the compounds. The amount of polar solvents, in particular water, may exist in a stoichiometric or unstoichiometric ratio. In case of stoichiometric solvates, e.g. hydrate, are possible hemi-, (semi-), mono-, sesqui-, di-, tri-, tetra-, penta- etc. solvates or hydrates, respectively.

As used herein, the term *"in vivo* hydrolysable ester" is understood as meaning an *in vivo* hydrolysable ester of a compound of formula (I) containing a carboxy or hydroxyl group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, e.g. methoxymethyl, C₁-C₆ alkanoyloxymethyl esters, e.g. pivaloyloxymethyl, phthalidyl esters, C₃-C₈ cycloalkoxy-carbonyloxy-C₁-C₆ alkyl esters, e.g. 1-cyclohexylcarbonyloxyethyl ; 1,3-dioxolen-2-onylmethyl esters, e.g. 5-methyl-1,3-dioxolen-2-onylmethyl ; and C₁-C₆-alkoxycarbonyloxyethyl esters, e.g. 1-methoxycarbonyloxyethyl, and may be formed at any carboxy group in the compounds of this invention. An *in vivo* hydrolysable ester of a compound of formula (I) containing a hydroxyl group includes inorganic esters such as phosphate esters and [alpha]-acyloxyalkyl ethers and related compounds which as a result of the *in vivo* hydrolysis of the ester breakdown to give the parent hydroxyl group. Examples of [alpha]-acyloxyalkyl ethers include acetoxymethoxy and 2,2-dimethylpropionyloxymethoxy. A selection of *in vivo* hydrolysable ester forming groups for hydroxyl include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

A further embodiment of the present invention relates to the use of a compound of general formula A, B as mentioned *infra* for the preparation of a compound of general formula (I) as defined *supra.*

The compounds of the present invention can be used in treating diseases of dysregulated vascular growth or diseases which are accompanied with dysregulated vascular growth. Especially, the compounds effectively interfere with Tie2 signalling. The compounds of the present invention show selective inhibition of Tie2 kinase vs. e.g. CDK2 and Aurora C kinase. By primarily targeting endothelial cell-specific kinases, compounds of the present invention may have an important advantage over prior art substances by reducing side effects which may result from interfering with signalling networks in non-endothelial cells. This effect can therefore allow prolonged treatment of patients with the compounds of the present invention offering good tolerability and high anti-angiogenic efficacy, where persistent angiogenesis plays a pathologic role.

Therefore, another aspect of the present invention is a use of the compound of general formula (I) described *supra* for manufacturing a pharmaceutical composition for the treatment of diseases of dysregulated vascular growth or of diseases which are accompanied with dysregulated vascular growth.

Preferably, the use is in the treatment of diseases, wherein the diseases are tumours and/or metastases thereof.

Another preferred use is in the treatment of diseases, wherein the diseases are retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

A further use is in the treatment of diseases, wherein the diseases are coronary and peripheral artery disease.

Another use is in the treatment of diseases, wherein the diseases are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

Yet another aspect of the invention is a method of treating a disease of dysregulated vascular growth or diseases which are accompanied with dysregulated vascular growth, by administering an effective amount of a compound of general formula (I) described *supra.*

Preferably, the diseases of said method are tumours and/or metastases thereof.

Also, the diseases of said method are retinopathy, other angiogenesis dependent diseases of the eye, in particular cornea transplant rejection or age-related macular degeneration, e.g. rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

Further, the disease of the method is coronary and peripheral artery disease.

Other diseases of the method are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

The compounds of the present invention can thus be applied for the treatment of diseases accompanied by neoangiogenesis. This holds principally for all solid tumours, e.g. breast, colon, renal, lung and/or brain tumours or metastases thereof and can be extended to a broad range of diseases, where pathologic angiogenesis is persistent. This applies for diseases with inflammatory association, diseases associated with oedema of various forms and diseases associated with stromal proliferation and pathologic stromal reactions broadly. Particularly suited is the treatment for gynaecological diseases where inhibition of angiogenic, inflammatory and stromal processes with pathologic character can be inhibited. The treatment is therefore an addition to the existing armament to treat diseases associated with neoangiogenesis.

The compounds of the present invention can be used in particular in therapy and prevention of tumour growth and metastases, especially in solid tumours of all indications and stages with or without pre-treatment if the tumour growth is accompanied with persistent angiogenesis. However, this is not restricted to tumour therapy but is also of great value for the treatment of other diseases with dysregulated vascular growth. This includes retinopathy and other angiogenesis dependent diseases of the eye (*e.g*. cornea transplant rejection, age-related macular degeneration), rheumatoid arthritis, and other inflammatory diseases associated with angiogenesis such as psoriasis, delayed type hypersensitivity, contact dermatitis, asthma, multiple sclerosis, restenosis, pulmonary hypertension, stroke and inflammatory diseases of the bowel, such as Crohn's disease. This includes coronary and peripheral artery disease. This can be applied for disease states such as ascites, oedema, such as brain tumour associated oedema, high altitude trauma, hypoxia induced cerebral oedema, pulmonary oedema and macular oedema or oedema following burns and trauma. Furthermore, this is useful for chronic lung disease, adult respiratory distress syndrome. Also for bone resorption and for benign proliferating diseases such as myoma, benign prostate hyperplasia and wound healing for the reduction of scar formation. This is therapeutically valuable for the treatment of diseases, where deposition of fibrin or extracellular matrix is an issue and stroma proliferation is accelerated (e.g. fibrosis, cirrhosis, carpal tunnel syndrome etc). In addition this can be used for the reduction of scar formation during regeneration of damaged nerves, permitting the reconnection of axons. Further uses are endometriosis, pre-eclampsia, postmenopausal bleeding and ovarian hyperstimulation.

Another aspect of the present invention is a pharmaceutical composition which contains a compound of Formula (I) or pharmaceutically acceptable salts thereof, N-oxides, solvates, hydrates, isomers or mixtures of isomers thereof, in admixture with one or more suitable excipients. This composition is particularly suited for the treatment of diseases of dysregulated vascular growth or of diseases which are accompanied with dysregulated vascular growth as explained above.

In order that the compounds of the present invention be used as pharmaceutical products, the compounds or mixtures thereof may be provided in a pharmaceutical composition, which, as well as the compounds of the present invention for enteral, oral or parenteral application contain suitably pharmaceutically acceptable organic or inorganic inert base material, e.g. purified water, gelatin, gum Arabic, lactate, starch, magnesium stearate, talcum, vegetable oils, polyalkylenglycol, *etc*.

The pharmaceutical compositions of the present invention may be provided in a solid form, e.g. as tablets, dragées, suppositories, capsules or in liquid form, e.g. as a solution, suspension or emulsion. The pharmaceutical composition may additionally contain auxiliary substances, e.g. preservatives, stabilisers, wetting agents or emulsifiers, salts for adjusting the osmotic pressure or buffers.

For parenteral applications, (including intravenous, subcutaneous, intramuscular, intravascular or infusion), sterile injection solutions or suspensions are preferred, especially aqueous solutions of the compounds in polyhydroxyethoxy containing castor oil.

The pharmaceutical compositions of the present invention may further contain surface active agents, e.g. salts of gallenic acid, phosphorlipids of animal or vegetable origin, mixtures thereof and liposomes and parts thereof.

For oral application tablets, dragées or capsules with talcum and/or hydrocarbon-containing carriers and binders, e.g. lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 to 1,500 mg. A dose can be administered as unit dose or in part thereof and distributed over the day. Accordingly the optimum dosage may be determined by the practitioner who is treating any particular patient.

It is possible for compounds of general formula(I) of the present invention to be used alone or, indeed in combination with one or more further drugs, particularly anti-cancer drugs or compositions thereof. Particularly, it is possible for said combination to be a single pharmaceutical composition entity, e.g. a single pharmaceutical formulation containing one or more compounds according to general formula(I) together with one or more further drugs, particularly anti-cancer drugs, or in a form, e.g. a "kit of parts", which comprises, for example, a first distinct part which contains one or more compounds according to general formula I, and one or more further distinct parts each containing one or more further drugs, particularly anti-cancer drugs. More particularly, said first distinct part may be used concomitantly with said one or more further distinct parts, or sequentially.

Another aspect of the present invention is a method which may be used for preparing the compounds according to the present invention.

The following Table lists the abbreviations used in this paragraph and in the Examples section as far as they are not explained within the text body.

| Abbreviation | Meaning |
|---|---|
| Ac | acetyl |
| Boc | *tert*-butyloxycarbonyl |
| br | broad |
| c- | cyclo- |
| Cl | chemical ionisation |
| d | doublet |
| dd | doublet of doublet |
| DCM | dichloromethane |
| DIPEA | N,N-diisopropylethyl amine |
| DMAP | N,N-dimethylaminopyridine |
| DMF | N, N-dimethylformamide |
| DMSO | dimethyl sulfoxide |
| eq. | equivalent |
| ESI | electrospray ionisation |
| GP | general procedure |
| HPLC | high performance liquid chromatography |
| LC-MS | liquid chromatography mass spectrometry |
| m | multiplet |
| mc | centred multiplet |
| MS | mass spectrometry |
| MTBE | methyl-tert-butyl ether |
| NMR | nuclear magnetic resonance spectroscopy : chemical shifts (δ) are given in ppm. |
| Pg | protecting group |
| POPd | dihydrogen dichlorobis(di-*tert*-butyl phosphinito-kP)palladate(2); CombiPhos Catalysts, Inc. |
| q | quartet |
| rf | at reflux |
| r.t. or rt | room temperature |
| s | singlet |
| sept. | septet |
| t | triplet |
| T3P | 1-propanephosphonic acid cyclic anhydride |
| TEA | triethylamine |
| TFA | trifluoroacetic acid |
| THF | tetrahydrofuran |
| TLC | thin-layer chromatography |

NMR peak forms are stated as they appear in the spectra, possible higher order effects have not been considered. Chemical names were generated using AutoNom2000 as implemented in MDL ISIS Draw.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be stirred out using a suitable solvent. In some cases, the compounds may be purified by chromatography, particularly flash column chromatography, using for example prepacked silica gel cartridges, e.g. from Separtis such as Isolute^{®} Flash silica gel or Isolute^{®} Flash NH₂ silica gel in combination with a Flashmaster II autopurifier (Argonaut/Biotage) and eluants such as gradients of hexane/EtOAc or DCM/ethanol. In some cases, the compounds may be purified by preparative HPLC using for example a Waters autopurifier equipped with a diode array detector and/or on-line electrospray ionization mass spectrometer in combination with a suitable prepacked reverse phase column and eluants such as gradients of water and acetonitrile which may contain additives such as trifluoroacetic acid or aqueous ammonia.

The following schemes and general procedures illustrate general synthetic routes to the compounds of general formula I of the invention and are not intended to be limiting. Specific examples are described in the subsequent paragraphs.

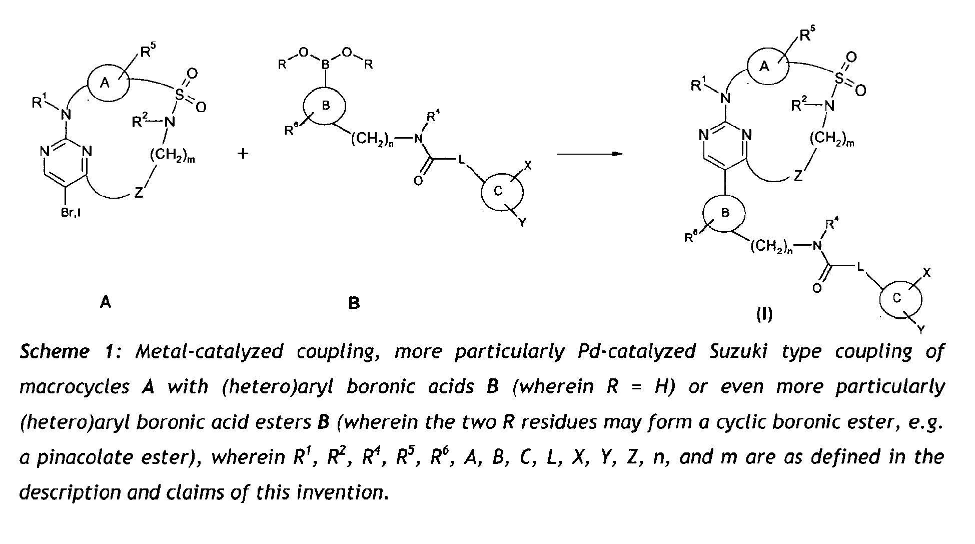

The compounds of the present invention can be prepared starting from halogenated macrocycles **(A)** by metal-catalyzed coupling reactions with e.g. (hetero)aryl boronic acids or their respective boronate esters **(B, *Scheme 1*)**. More particularly, compounds of the present invention can be prepared starting from brominated or iodinated macrocycles **(A)** by Pd-catalyzed Suzuki-type coupling reactions with (hetero)aryl boronic acids **(B)** or even more particularly with their respective boronic acid esters **(B**, e.g. a pinacolate ester). Transition metal-catalyzed couplings of heteroaryl bromides and iodides with (hetero)aryl boronic acids or aryl boronic acid esters are well known to the person skilled in the art. Various catalyst/ligand/base/solvent combinations have been published in the scientific literature (Tetrahedron 2005, 61, 5131 and references cited therein; Eur. J. Org. Chem. 2006, 1917 and references cited therein; Eur. J. Org. Chem. 2006, 2063 and references cited therein), which allow a fine-tuning of the required reaction conditions in order to allow for a broad set of additional functional groups on both coupling partners.

The synthesis of the required halogenated macrocycles **(A)** is described in WO 2004026881. The syntheses of two macrocycles of the formula A, in which R¹, R², R³ and R⁵ are standing for hydrogen, A is a meta-connected phenylene, Z is NH and m is the integer of 3, are herein exemplified as ***Preparation Example A**,* particularly with respect to the brominated macrocycle **A**, and even more particularly as ***Preparation Example B,*** with respect to the iodinated macrocycle **A**. In addition to said two macrocycles, WO2004026881 specifically discloses a variety of procedures suitable for the preparation of various intermediates of the formula A, featuring various m, R⁵, R³, Z and A groups. These compounds may in turn be used for further modifications such as interconversions of R¹, R², R³ groups and more particularly interconversions of R⁵ groups. For example, macrocyclic compounds of general formula A, wherein R⁵ stands for an amino group, may be further derivatized by standard amine transformations known to the person skilled in the art, including, but not limited to, e.g. alkylations, acylations, sulfonylations, or urea formations.

The boronic acids and their respective boronic acid esters **(B)** (e.g. their respective pinacolate esters) needed for the above mentioned coupling reactions can be prepared by amide coupling of accordingly substituted anilines (or benzylic amines or higher homologs) and carboxylic acids **(*Scheme* 2).** Many methods for amide formations are extractable from the scientific literature for the person skilled in the art including, but not limited to, pre-formation of the more reactive carboxylic acid chloride (by reaction of carboxylic acids with e.g. thionyl chloride or sulfuryl chloride or oxalyl chloride), or *in situ* activation of the carboxylic acid in the presence of the aniline (or benzylic amine) by reaction with coupling reagents e.g. dicyclohexylcarbodiimide (DCC)/dimethylaminopyridine (DMAP), ethyldimethylaminopropylcarbodiimide (EDC)/DMAP, N,N'-carbonyldiimidazole (CDI), or T3P and others known to the person skilled in the art. The boronic acid or boronic acid ester moiety (e.g. a pinacolate ester) can either be present in the commercially available starting materials or introduced before or after amide coupling. Boronic acids or boronic acid esters can be introduced into aryl or heteroaryl compounds *inter alia* by substituting halogen atoms. This substitution can be accomplished by metalation followed by electrophilic borylation (Org. Biomol. Chem. 2004, 2, 852) or by direct Pd- or Cu-catalyzed borylation (Synlett 2003, 1204 and references cited therein; Org. Lett. 2006, 8, 261). Alternatively, aromatic CH bonds can be borylated employing lr catalysts (see: J. Am. Chem. Soc. 2005, 127, 10539 and references cited therein). Interconversion of boronic acids into the respective esters (e.g. their pinacolate esters) can be accomplished under standard conditions (for example by treatment with pinacol in EtOH at r.t.).

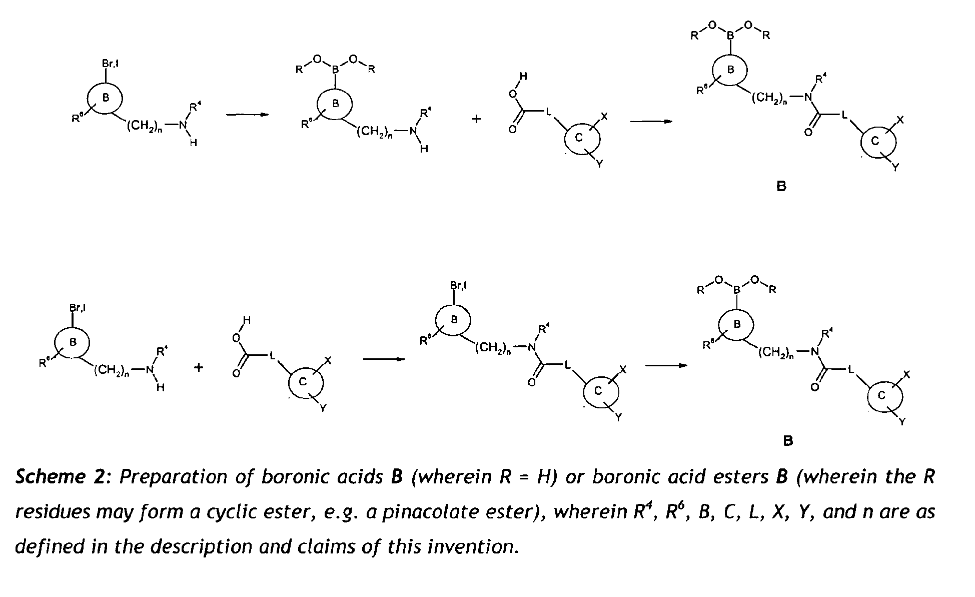

The carboxylic acids required for the above described amide coupling reactions are either commercially available or are accessible from commercially available carboxylic esters or nitriles. Alternatively, (hetero)aryls bearing a methylenenitrile substituent are easily accessible from the respective halides via nucleophilic substitution reactions (e.g. employing KCN and a catalytic amount of KI in EtOH/H₂O). Incorporation of additional functionality into commercially available starting materials can be accomplished by a multitude of aromatic transformation reactions known to the person skilled in the art, including, but not limited to, e.g. electrophilic halogenations, electrophilic nitrations, Friedel-Crafts acylations, nucleophilic displacement of fluorine by oxygen nucleophiles and transformation of (hetero)aryl carboxylic acids into amides and subsequent reduction into benzylic amines, wherein the latter two methods are of particular relevance for the introduction of ether and/or aminomethylene side chains.

Benzylic nitriles and esters (and heteroaryl analogs thereof) can be efficiently alkylated at the benzylic position under basic conditions and subsequently hydrolyzed to the corresponding alkylated acids. Conditions for α-alkylations of nitriles and esters include, but are not limited to, the use of alkyl bromides or alkyl iodides as electrophiles under basic conditions in the presence or absence of a phase-transfer catalyst in a mono- or biphasic solvent system. Particularly, by using excess alkyl iodides as electrophilic species α,α-dialkylated nitriles are accessible. More particularly, by using 1,ω-dihaloalkyls as electrophiles cycloalkyl moieties can be installed at the benzylic position of nitriles and esters *(*J. Med. Chem. 1975, 18, 144; WO2003022852). The hydrolysis of nitriles to yield carboxylic acids can be accomplished, as known to the person skilled in the art, under acid or base-mediated conditions. As an exemplification of the described general synthetic route toward functionalized carboxylic acids the more particular synthesis of substituted 1-phenylcyclopropylcarboxylic acids is described in the following scheme **(*Scheme 3*).**

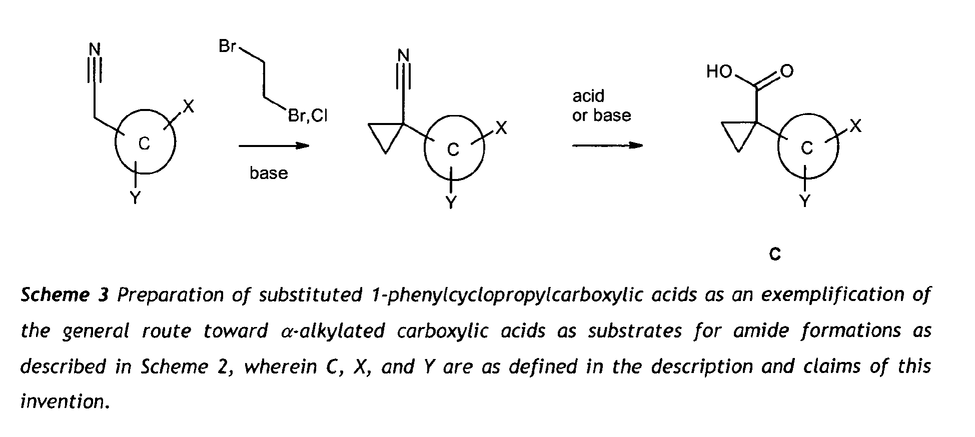

The above described general route to (hetero)aryl-cyclopropyl carboxylic acid is also applicable for the synthesis of the analogous higher homologs of (hetero)aryl-cycloalkyl carboxylic acids.

The substituents R¹, R², R³, R⁴, R⁵, R⁶, X and Y in compounds of general formula (I) and in compounds of general formula A, B, and may be further modified.These modifications can be such as e.g. the introduction of protecting groups, cleavage of protecting groups, reduction or oxidation of functional groups, alkylations, substitution or other reactions. Appropiate protecting groups and their introduction and cleavage are well-known to the person skilled in the art (see for example T.W. Greene and P.G.M. Wuts in Protective Groups in Organic Synthesis, 3rd edition, Wiley 1999).

### Examples

In the subsequent paragraphs general procedures for the synthesis of the below mentioned specific example compounds are summarized.

### General Procedure 1a (GP1a): Suzuki coupling conditions A

A solution of the respective macrocyclic halide in DMF (8 mL per mmol halide) was treated with the respective organoboron compound (1.25 eq.), K₂CO₃ (2.5 eq., 2 M aqueous solution), and POPd (2.5-5 mol-%) at room temperature. The stirred resulting mixture was placed into an oil bath preheated to 105 °C. The reaction progress was monitored by TLC, and in case of incomplete turnover of the macrocyclic halide after 2h additional portions of POPd and the organoboron compound were added followed by additional stirring at 105 °C. After complete turnover, the reaction mixture was diluted with ethyl acetate and quenched with water. The layers were separated, the aqueous layer extracted with EtOAc twice. The combined organic layers were washed with brine, dried and concentrated in vacuo. The crude product was purified by column chromatography, followed optionally by trituration with methanol and/or preparative HPLC to yield the analytically pure products.

### General Procedure 1b (GP1b): Suzuki coupling conditions B

The respective macrocyclic halide (1 eq.), the respective boronic acid pinacolate ester (1.4 eq.), LiCl (2.8 eq.), and Pd(PPh₃)₄ (8 mol%) were placed into a flask under an atmosphere of argon. Toluene (20 mL per mmol halide) and EtOH (20 mL per mmol halide) were added by syringe followed by an aqueous solution of sodium carbonate (1M, 2.5 eq.). The resulting slurry or solution was placed into an oilbath preheated to 90 °C and stirred until TLC indicated complete turnover of the starting halide. The reaction mixture was then diluted with ethyl acetate and quenched with water. The layers were separated, the aqueous layer extracted with EtOAc twice. The combined organic layers were washed with brine, dried and concentrated in vacuo. The crude product was purified by column chromatography, followed optionally by trituration with methanol and/or preparative HPLC to yield the analytically pure products.

### General Procedure 2a (GP 2a): Cyclopropanation conditions A

To a stirred mixture of the respective benzyl cyanide (1.0 eq.), triethylbenzylammonium chloride (2 mol%) and 1,2-dibromoethane (2 eq.) was added dropwise 50% aq. NaOH solution (6-7 eq.). After complete addition the reaction is stirred at 40-50 °C until complete turnover. The reaction mixture was then diluted with water, the organic products extracted with benzene, the combined organic extracts were washed with 15% aq. HCl and subsequently with water, dried and concentrated in vacuo. The crude products were in general used for the subsequent nitrile hydrolysis without further purification.

### General Procedure 2b (GP 2b): Cyclopropanation conditions B

To a stirred mixture of the respective benzyl cyanide (1.0 eq.), triethylbenzylammonium chloride (2 to 4 mol%) and 1-bromo-2-chloroethane (2 to 5 eq.) was added dropwise 50% aq. NaOH solution (6-8 eq.). After complete addition the reaction is stirred at 40-50 °C until complete turnover. The reaction mixture was then diluted with water, the organic products extracted with benzene, the combined organic extracts were washed with 5% aq. HCl and subsequently with water, dried and concentrated in vacuo. The crude products were in general used for the subsequent nitrile hydrolysis without further purification.

### General Procedure 3a (GP 3a): Nitrile hydrolysis conditions A

To a stirred mixture of the respective substituted phenylcyclopropanecarbonitrile (1.0 eq.) in EtOH (- 7 mL per mmol nitrile) was added a 10% aq. NaOH solution (2.0 to 2.5 eq.) and the resulting mixture was refluxed for 24h, after which the mixture was concentrated in vacuo and the residual aqueous phase was extracted with MTBE. The aqueous layer was then acidified with concentrated HCl under ice cooling and the thus formed precipitate filtered off. The filter cake was washed with water and dried to yield the analytically pure carboxylic acid.

### General Procedure 3b (GP 3b): Nitrile hydrolysis conditions B

The respective phenylcyclopropanecarbonitrile (1.0 eq.) together with concentrated HCl (1 mL per mmol nitrile) was stirred in a pressure tube at 100 °C for 16h. The mixture was poured on ice, extracted with dichloromethane, the organic extract was washed with water and brine and dried. After concentration in vacuo the carboxylic acid crystallized upon standing.

### General Procedure 4a (GP 4a): Amide formation conditions A

To a solution of the respective carboxylic acid (1 eq.) in dichloromethane (2.5 mL per mmol carboxylic acid), which contained 2 drops of DMF, a solution of oxalyl chloride (3.5 eq.) in dichloromethane (1 mL per mmol carboxylic acid) was added dropwise at 0 °C. After complete addition the mixture was gradually warmed to room temperature and stirring was continued for 16h. Subsequently, excess oxalyl chloride and solvents were removed in vacuo and the crude carboxylic acid chloride was used without further purification.

The crude carboxylic acid chloride (1 eq.) was dissolved in pyridine (2.5 mL per mmol carboxylic acid chloride). A solution of the respective aniline (or benzylamine or higher homolog; 1.0 eq) in pyridine (1.5 mL per mmol) was added dropwise at 0 °C to this solution and stirring was continued at 40 °C for 4h. After cooling to room temperature, the mixture was quenched by addition of water and extracted with MTBE twice. The combined organic layers were washed with 5% aq. HCl, with water and brine, dried and concentrated in vacuo. The residual oil was treated with MTBE/hexane (1:1). The so formed precipitate was filtered off, washed with ice-cold hexane and dried to yield the respective amide in analytically pure form.

### General Procedure 4b (GP 4b): Amide formation conditions B

The respective carboxylic acid was treated with thionyl chloride (6 eq.) and stirred at 38 °C for three days. Excess thionyl chloride was removed in vacuo and the resulting crude carboxylic acid chloride was used without further purification steps.

The respective aniline (or benzylamine or higher homolog; 1.0 eq.) and the respective crude carboxylic acid chloride (1.5 eq.) were stirred in pyridine (0.2 M) at room temperatur for 2 days. The volatiles were removed in vacuo, the residue was taken up in dichloromethane and the desired amides were crystallized by addition of hexane.

### General Procedure 4c (GP 4c): Amide formation conditions C

The respective carboxylic acid was treated with thionyl chloride (6 eq.) and stirred at 38 °C for three days. Excess thionyl chloride was removed in vacuo and the resulting crude carboxylic acid chloride was used without further purification steps.

The crude carboxylic acid chloride (1 eq.) was dissolved in DCM (2 mL per mmol carboxylic acid chloride) and treated with triethylamine (2 eq.). A solution of the respective aniline (or benzylamine or higher homolog; 1.0 eq) in DCM was added dropwise at 0 °C to this solution and stirring was continued at room temperature for 36h. The organic mixture was subsequently washed with 5% aq. HCl and the aqueous layer was re-extracted with DCM. The combined organic layers were washed with water, dried and concentrated in vacuo. The resulting oily residue was treated with methanol and the precipitate thus formed was filtered off, washed with hexane and dried to yield the target amide.

### Preparation Example A:

### Production of 6-Bromo-13-thia-2,4,8,12,19-pentaaza-tricyclo[12.3.1.1^{3,7}]-nonadeca-1(18),3(19),4,6,14,16-hexaene 13,13-dioxide

### Step 1

### 3-Amino-N-[3-(5-bromo-2-chloro-pyrimidin-4-ylamino)-propyl]-benzenesulfonamide

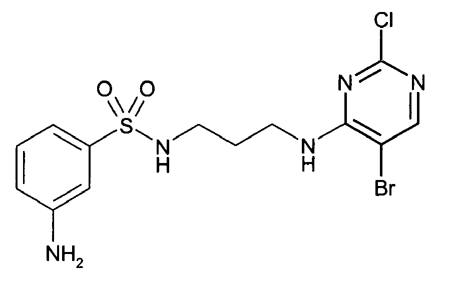

A solution of 1.35 g (2.99 mmol) of N-[3-(5-bromo-2-chloro-pyrimidin-4-ylamino)-propyl]-3-nitro-benzenesulfonamide in 100 ml of tetrahydrofuran was mixed under argon at room temperature with 15 ml of a 15% solution of Ti(III)Cl₃ in about 10% hydrochloric acid. After 17 hours, another 1 ml of the Ti(III)Cl₃ solution was added and the mixture was stirred for another 3 hours. 1 N NaOH solution was then added to the reaction mixture until pH > 10 and the slurry was suction filtered. The filter cake was washed 2x with 100 ml of ethyl acetate/MeOH (3:2). The filtrate was concentrated in vacuo and extracted with ethyl acetate (4x). The combined organic layers were washed with NaCl solution, dried (Na₂SO₄), filtered and concentrated by evaporation. The remaining residue was further purified by column chromatography (dichloromethane/ MeOH 95:5) to yield 624 mg (1.48 mmol, 49% yield) of the analytically pure product.

¹H-NMR (DMSO): 8.21 (s, 1 H), 7.63 (t, 1 H), 7.38 (t, 1 H), 7.13 (t, 1 H), 6.97 (m, 1 H), 6.83 (m, 1 H), 6.71 (m, 1 H), 5.53 (s, 2H), 3.30 (m, 2H), 2.75 (m, 2H), 1.65 (m, 2H).

### Step 2

### 6-Bromo-13-thia-2,4,8,12,19-pentaaza-tricyclo[12.3.1.1^{3,7}]-nonadeca-1(18),3(19),4,6,14,16-hexaene 13,13-dioxide

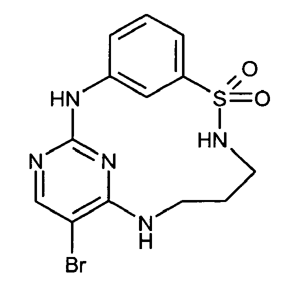

A solution of 200 mg (0.48 mmol) of 3-amino-N-[3-(5-bromo-2-chloro-pyrimidin-4-ylamino)-propyl]-benzenesulfonamide in acetonitrile/water/2-butanol (9.0 ml/1.0 ml/0.3 ml) was added via a syringe pump within 2.5 hours to a refluxing mixture of acetonitrile/water/4 N HCl in dioxane (45 ml/5 ml/0.6 ml). After completion of addition the resulting mixture is refluxed for another 3 hours. The reaction mixture was then allowed to reach room temperature and additional product precipitated upon standing at room temperature over night. The precipitate was filtered off, washed with water and dried in vacuum yielding 112 mg (0.31 mmol) of a white solid. The filtrate was concentrated in vacuo. The precipitate that is formed was again filtered, washed with water and dried to yield another 45 mg of the product.

In total 157 mg of the product (0.41 mmol, 85% yield) were obtained in an analytically pure form.

¹H-NMR (DMSO): 10.45 (s, 1 H), 9.07 (s, 1 H), 8.35 (br, 1 H), 8.18 (s, 1 H), 7.78 (t, 1 H), 7.45 (m, 2H), 7.32 (m, 1 H), 3.44 (m, 2H), 3.28 (m, 2H), 1.82 (m, 2H).
MS (ESI): [M+H]⁺ = 384 .

### Preparation Example B:

### Production of 6-Iodo-13-thia-2,4,8,12,19-pentaaza-thricyclo[12.3.1.1^{3,7}]nonadeca-1(18),3(19),4,6,14,16-hexaene 13,13-dioxide

### Step 1

### 3-Amino-N-[3-(2-chloro-5-iodo-pyrimidin-4-ylamino)-propyl]-benzenesulfonamide

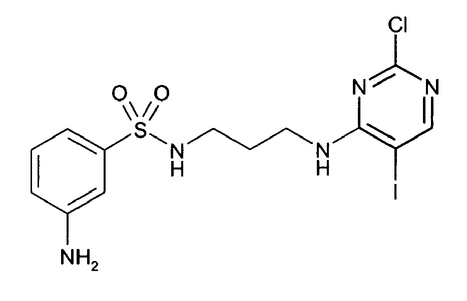

A solution of 9.95 g (20.0 mmol) of N-[3-(2-chloro-5-iodo-pyrimidin-4-ylamino)-propyl]-3-nitro-benzenesulfonamide in 660 mL of tetrahydrofuran was mixed under argon at room temperature with 100 mL of a 15% solution of Ti(III)Cl₃ in about 10% hydrochloric acid. After 2 hours, another 7 mL of the Ti(III)Cl₃ solution was added to the reaction mixture and stirring was continued for one hour. 1 N NaOH solution was then added to the reaction mixture until pH > 12 and the slurry was suction filtered over Celite. The filtrate was extracted with ethyl acetate (3x 400 mL), the combined organic layers were washed with brine (200 mL), and concentrated in vacuo. The filter cake was rewashed 4x with 500 ml of ethyl acetate/MeOH (3:2), followed by evaporation of the resulting washing fractions. The residues were combined and purified by column chromatography over silica (dichloromethane/ethyl acetate) to give 5.42 g (58 % yield) of the target compound.

¹H-NMR (DMSO, 300 MHz): 8.31 (s, 1 H), 7.39 (t, 1 H), 7.27 (t, 1 H), 7.16 (t, 1 H), 6.95 - 7.01 (m, 1H), 6.82 - 6.88 (m, 1H), 6.68 - 6.76 (m, 1H), 5.53 (s, 2H), 3.27 - 3.39 (m, 2H), 2.68 - 2.82 (m, 2H), 1.64 (mc, 2H).
ESI-MS: [M+H⁺] = 468 (³⁵Cl signal) + 470 (³⁷Cl signal)

### Step 2:

### 6-Iodo-13-thia-2,4,8, 12,19-pentaaza-tricyclo[12.3.1.1^{3,7}]nonadeca-1(18),3(19),4,6,14,16-hexaene 13,13-dioxide

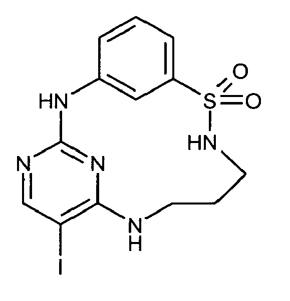

A solution of 2.34 g (5.00 mmol) of 3-amino-N-[3-(5-iodo-2-chloro-pyrimidin-4-ylamino)-propyl]-benzenesulfonamide in acetonitrile/water/2-butanol (94 mL/10.4 mL/3.1 mL) was added via a syringe pump within 3 hours to a refluxing mixture of acetonitrile/water/4 N HCl in dioxane (470 mL/52 mL/6.2 mL). After completion of addition the resulting mixture is refluxed for another 3 hours. The reaction mixture was then allowed to reach room temperature and additional product precipitated upon standing at room temperature over night. The precipitate was filtered off, washed with water and dried in vacuum yielding 1.71 g (79% yield) of a white solid.

¹H-NMR (DMSO, 300 MHz): 10.81 (s, 1H), 9.02 (s, 1H), 8.30 - 8.38 (m, 1H), 8.27 (s, 1 H), 7.82 (t, 1 H), 7.43 - 7.56 (m, 2H), 7.29 - 7.40 (m, 1 H), 3.38 - 3.52 (m, 2H), 3.21 - 3.36 (m, 2H), 1.72 - 1.90 (m, 2H).
ESI-MS: [M+H⁺] = 432.

The following boronic acid pinacolate esters were prepared by amide formation from 4-(4,4,5,5-tetramethyl-1,3,2-dioxaborolan-2-yl)-aniline with the respective carboxylic acid by applying general procedure GP 4a or GP 4b (compare to **Scheme 2).** The respective carboxylic acids were either commercially available (**Entries 1, 4, 6, 13**) or prepared from the respective benzylic nitriles by cyclopropanation (general procedure GP 2a or GP 2b) followed by nitrile hydrolysis (general procedure GP 3a or GP 3b; compare to **Scheme 3)**. The specifically applied general procedures are listed in the table along with the respective structure.

| **Entry** | **Structure** | **Name** | **Analytical data** |
|---|---|---|---|
| **1** | 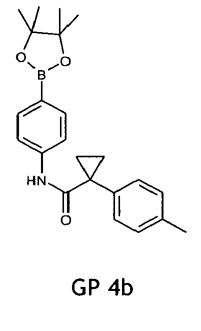 | 1-p-Tolylcyclopropane-carboxylic acid [4-(4,4,5,5-tetramethyl-[1,3,2]dioxaborolan-2-yl)-phenyl]-amide | ¹H-NMR (DMSO, 300 MHz): 8.90 (s, 1 H); 7.48-7.52 (m, 4 H); 7.24-7.27 (m, 2 H); 7.10-7.17 (m, 2 H); 2.26 (s, 3 H); 1.37-1.40 (m, 2 H); 1. 23 (s, 12 H); 1.01 - 1.05 (m, 2 H). MS (ESI): [M+H]⁺ = 378 |
| **2** | 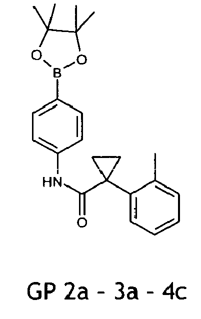 | 1-o-Tolyl-cyclopropane-carboxylic acid [4-(4,4,5,5-tetramethyl-[1,3,2]dioxaborolan-2-yl)-phenyl]-amide | ¹H-NMR (DMSO, 300 MHz): 8.37 (s, 1 H); 7.50 (d, 2 H); 7.42 (d, 2 H); 7.35 - 7.38 (m, 1 H); 7.15 - 7.24 (m, 3 H); 2.26 (s, 3 H); 1.50 - 1.54 (m, 2 H); 1.22 (s, 12 H); 1.02- 1.06 (m, 2 H). |
| **3** | 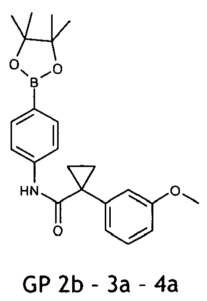 | 1-(3-Methoxy-phenyl)-cyclopropane-carboxylic acid [4-(4,4,5,5-tetramethyl-[1,3,2]dioxaborolan-2-yl)-phenyl]-amide | ¹H-NMR (CDCl₃, 300 MHz): 8.65 (s, 1 H); 7.70 (d, 2 H); 7.20 - 7.40 (m, 5 H); 7.10 (d, 1 H); 7.02 (s, 1 H); 6.90 (d, 1 H); 3.85 (s, 3H); 1.70 - 1.74 (m, 2 H); 1.32 (S, 12 H); 1.15 - 1.20 (m, 2 H). |
| **4** | 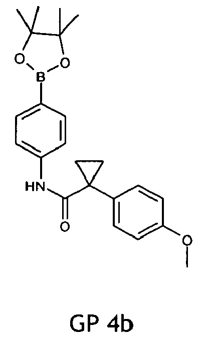 | 1-(4-Methoxy-phenyl)-cyclopropane-carboxylic acid [4-(4,4,5,5-tetramethyl-[1,3,2]dioxaborolan-2-yl)-phenyl]-amide | ¹H-NMR (DMSO, 300 MHz): 8.78 (s, 1 H); 7.48 - 7.52 (m, 4 H); 7.29 - 7.34 (m, 2 H); 6.86 - 6.91 (m, 2 H); 3.72 (s, 3 H); 1.36 - 1.40 (m, 2 H); 1.23 (s, 12 H); 0.99 - 1.03(m, 2H). MS (ESI): [M+H]⁺ = 394. |
| **5** | 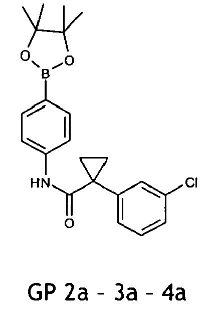 | 1-(3-Chloro-phenyl)-cyclopropane-carboxylic acid [4-(4,4,5,5-tetramethyl-[1,3,2]dioxaborolan-2-yl)-phenyl]-amide | ¹H-NMR (DMSO, 300 MHz): 9.28 (s, 1 H); 7.54 (s, 4 H); 7.38 (s, 1 H); 7.28 - 7.35 (m, 3 H); 1.40 - 1.44 (m, 2 H); 1.23 (s, 12 H); 1.10-1.14(m,2H). |
| **6** | 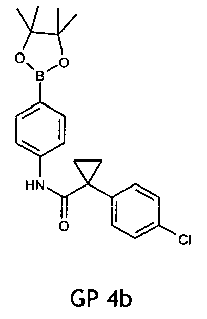 | 1-(4-Chloro-phenyl)-cyclopropane-carboxylic acid [4-(4,4,5,5-tetramethyl-[1,3,2]dioxaborolan-2-yl)-phenyl]-amide | ¹H-NMR (DMSO, 300 MHz): 9.14 (s, 1 H); 7.53 (br. s 4 H); 7.37 (br. s, 4 H); 1.41 - 1.44 (m, 2 H); 1.23 (s, 12 H); 1.06 - 1.09 (m, 2 H). MS (ESI): [M+H]⁺ = 398. |
| **7** | 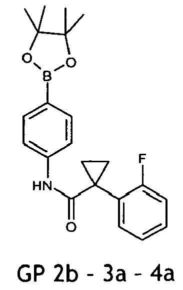 | 1-(2-Fluoro-phenyl)-cyclopropane-carboxylic acid [4-(4,4,5,5-tetramethyl-[1,3,2]dioxaborolan-2-yl)-phenyl]-amide | ¹H-NMR (CDCl₃, 300 MHz): 7.10 - 7.80 (m, 8 H); 1.70 - 1.85 (m, 2 H); 1.33 (s, 12 H); 1.15- 1.25 (m, 2 H). |
| **8** | 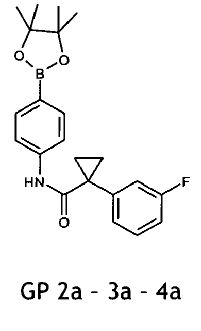 | 1-(3-Fluoro-phenyl)-cyclopropane-carboxylic acid [4-(4,4,5,5-tetramethyl-[1,3,2]dioxaborolan-2-yl)-phenyl]-amide | ¹H-NMR (DMSO, 300 MHz): 9.25 (s, 1 H); 7.51 - 7.57 (m, 4 H); 7.31 - 7.39 (m, 1 H); 7.04 - 7.20 (m, 3 H); 1.41 - 1.44 (m, 2 H); 1.23 (s, 12 H); 1.10 - 1.14 (m, 2 H). |
| **9** | 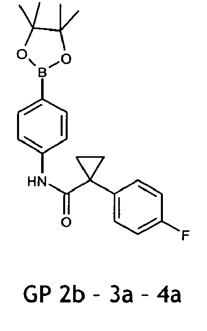 | 1-(4-Fluoro-phenyl)-cyclopropane-carboxylic acid [4-(4,4,5,5-tetramethyl-[1,3,2]dioxaborolan-2-yl)-phenyl]-amide | ¹H-NMR (DMSO, 300 MHz): 7.70 (D, 2 H); 7.46 - 7.54 (m, 2 H); 7.36 (d, 2 H); 7.29 (s, 1 H); 7.16 (t, 2 H); 1.65-1.80 (m, 2 H); 1.33 (s, 12 H); 1.15 - 1.25 (m, 2 H). |
| **10** | 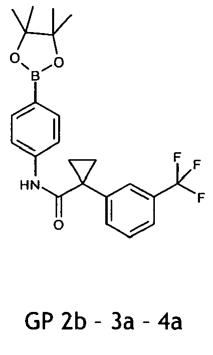 | 1-(3-Trifluoromethyl-phenyl)-cyclopropane-carboxylic acid [4-(4,4,5,5-tetramethyl-[1,3,2]dioxaborolan-2-yl)-phenyl]-amide | ¹H-NMR (CDCl₃, 300 MHz): 7.57 - 7.76 (m, 5 H); 7.34 (d, 2 H); 7.27 (s, 1 H); 6.94 (s, 1 H); 1.75 - 1.85 (m, 2 H); 1.30 (s, 12 H); 1.15 - 1.25 (m, 2 H). |
| **11** | 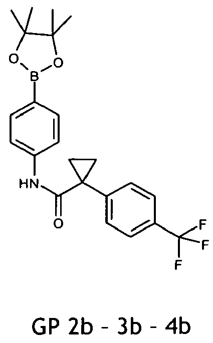 | 1-(4-Trifluoromethyl-phenyl)-cyclopropane-carboxylic acid [4-(4,4,5,5-tetramethyl-[1,3,2]dioxaborolan-2-yl)-phenyl]-amide | ¹H-NMR (DMSO, 300 MHz): 9.38 (br. s, 1 H); 7.66 (d, 2 H); 7.55 (s, 4 H); 7.54 (d, 2H); 1.47 - 1.50 (m, 2 H); 1.23 (s, 12 H); 1.14-1.18(m,2H). MS (ESI): [M+H]⁺ = 432. |
| **12** | 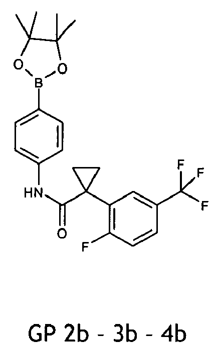 | 1-(2-Fluoro-5-trifluoromethyl-phenyl)-cyclopro-panecarboxylic acid [4-(4,4,5,5-tetramethyl-[1,3,2]dioxaborolan-2-yl)-phenyl]-amide | ¹H-NMR (DMSO, 300 MHz): 9.08 (br. s, 1 H); 7.76-7.83 (m, 2 H); 7.52 - 7. 59 (m, 4 H); 7.45 (t, 1H); 1.60 - 1.65 (m, 2 H); 1.28 (s, 12 H); 1.23 - 1.27 (m, 2 H). MS (ESI): [M+H]⁺ = 450 |
| **13** | 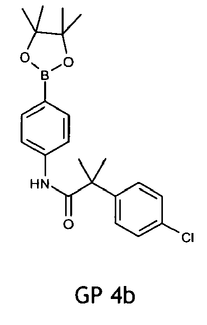 | 2-(4-Chloro-phenyl)-N-[4-(4,4,5,5-tetramethyl-[1,3,2]dioxaborolan-2-yl)-phenyl]-isobutyramide | ¹H-NMR (DMSO, 400MHz): 9.18 (s, 1 H); 7.53 - 7.60 (m, 4 H); 7.31- 7.38 (m, 4 H); 1.51 (s, 6 H); 1.23 (s, 12 H). |

In addition to those boronic acid pinalocate esters explicitely disclosed in the above table, a broad range of additional substitution patterns is accessible by applying the described general procedure GP 2a/2b, 3a/3b and 4a/4b to other nitriles and amine substrates. Of particular use as starting materials for these transformations are 4-(4,4,5,5-tetramethyl-[1,3,2]dioxaborolan-2-yl)-benzylamine (commercially available) and 2-fluoro-4-(4,4,5,5-tetramethyl-[1,3,2]dioxaborolan-2-yl)-phenylamine (synthesis described below).

### Preparation of 2-Fluoro-4-(4,4,5,5-tetramethyl-[1,3,2]dioxaborolan-2-yl)-phenylamine

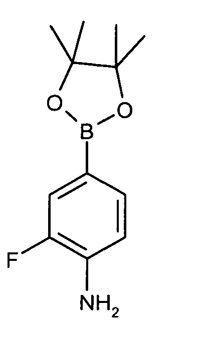

### Route A (Metallation)

### Step 1

50 g of 4-bromo-2-fluoroaniline (263 mmol) and 70 g of Boc₂O (321 mmol) were dissolved in tert-BuOH (140 mL) and stirred at 50 °C overnight. TLC indicated completeness of reaction. The solvent was mainly (-2/3) evaporated, then 150 mL of 50% MeOH was added and subsequently 15 mL of conc. NH₃. After 30 min stirring the oily lower layer was separated, washed with 50% MeOH, concentrated in vacuo and the product crystallized upon cooling. Then the product was filtered off, the filtrate was dissolved in benzene, extracted with 3% HCl, then evaporated and an additional portion of BOC-aniline crystallized by dilution with 75% EtOH (total yield: 55 g, 190 mmol, 72%).

¹H-NMR (DMSO, 300 MHz): 9.10 (s, 1 H); 7.58 (t, 1 H); 7.51 (dd, 1 H); 7.33 (dd, 1 H); 1.45(s,9H).

### Step 2

The solution of the product from step 1 (25 g, 86 mmol) in THF (400 mL), was cooled to -85 °C, and then treated dropwise with 83 mL of 2.5 M n-BuLi (208 mmol). The mixture was stirred for 1 h, and quenched with trimethylborate (27 g, 260 mmol) at -90 ° C. The viscous mixture was gradually warmed to rt, poured into 1 L of water, extracted with benzene (50 mL) and evaporated. The aqueous layer was neutralized with acetic acid, upon which the precipitated oil slowly started to crystallize. The precipitate was filtered off, washed with water and air-dried yielding 15g of the boronic acid. The aqueous filtrate was extracted with EtOAc, the organic layers were combined, dried and evaporated to dryness. Flash column chromatography (PhH - PhH:EtOH 3:1) yielded another batch of the boronic acid (1.2 g) improving the combined yield to 74% (64 mmol).
¹H-NMR (DMSO, 300 MHz): 9.00 (s, 1 H); 8.20 (br. s, 2 H); 7.62 (t, 1 H); 7.48 - 7.54 (m, 2 H); 1.50 (s, 9 H).

### Step 3

15 g of the boronic acid from step 2 (59 mmol) and 14 g pinacol (118 mmol) were stirred in MeOH at rt for 1 h. TLC indicated complete conversion, water (45 mL) was added to the reaction mixture, and the precipitated oil started crystallizing after trituration. The precipitate was filtered, washed with 70% MeOH, and dried (16 g, 48 mmol, yield: 81%).

¹H-NMR (DMSO, 300 MHz): 9.11 (s, 1 H); 7.73 (t, 1 H); 7.38 (d, 1 H); 7.28 (d, 1 H); 1.43 (s, 9 H); 1.25 (s, 12 H).

### Step 4

A solution of the BOC-derivative from step 3 (3.6 g, 10.6 mmol) in 35 mL DCM and 10 mL of 5N HCl in dioxane was stirred at 30 °C for 1.5 h. TLC indicated 80% conversion. Additional 5 mL of HCl/dioxane were added, and stirring was continued for 1 h. The solvent was evaporated, the residue was treated with water, neutralized with NaHCO₃ and extracted with benzene. The combined organic layers were washed with water, evaporated to dryness, and the resulting oil was triturated with hexane to yield 1.29 g (5.4 mmol) of the boronic acid pinacolate ester (51%).

¹H-NMR (DMSO, 300 MHz): 7.12 (dd, 1 H); 7.11 (dd, 1 H); 6.68 (t, 1 H); 5.53 (br. s, 2 H); 1.21 (S, 12 H).

### Route A (Pd-catalyzed borylation)

### Step 1

570 mg 4-bromo-2-fluoroaniline (3 mmol, 1 eq.), 1.14 g bis(pinacolato)diboron (4.5 mmol, 1.5 eq.), 883 mg KOAc (9 mmol, 3 eq.) and 245 mg PdCl₂(dppf)•CH₂Cl₂ (0.3 mmol, 0.1 eq.) were weighed into a dry (Schlenk) flask and set under an atmosphere of argon. 10.4 mL DMSO were added and the resulting reddish-purple solution was heated to 80 °C for 6.5 h. The mixture was diluted with EtOAc, quenched with water, and filtered through Celite. The layers were separated and the watery layer was extracted with EtOAc. The combined organic layers were washed with brine (2x), dried and concentrated in vacuo. The residue was purified by flash column chromatography to yield 661 mg of the desired product (90%), which contained pinacole as a slight impurity.

¹H-NMR (DMSO, 300 MHz): 7.12 (dd, 1 H); 7.11 (dd, 1 H); 6.68 (t, 1 H); 5.53 (br. s, 2 H); 1.21 (S, 12 H).

The following example compounds were prepared by Suzuki couplings according to the general procedure GP 1a or GP 1b from 6-Iodo-13-thia-2,4,8,12,19-pentaaza-tricyclo[12.3.1.1^{3,7}]nonadeca-1(18),3(19),4,6,14,16-hexaene 13,13-dioxide and the respective boronic acid pinacolate esters.

| **Example** | **Structure (Procedure)** | **Name** | **Analytical date** |
|---|---|---|---|
| **1** | 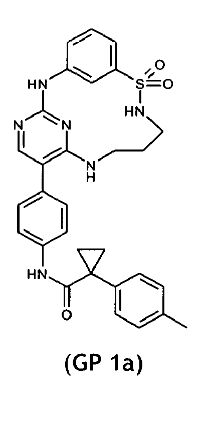 | 1-p-Tolyl-cyclopropane-carboxylic acid [4-(13,13-dioxo-13λ⁶-thia-2,4,8,12,19-pentaaza-tricyclo[12.3.1.1^{3,7}]nonadeca-1(18),3(19),4,6,14,16-hexaen-6-yl)-phenyl]-amide | ¹H-NMR (DMSO, 400 MHz): 9.55 (s, 1 H); 9.41 (s, 1 H); 9.06 (s, 1 H); 7.71 - 7.74 (m, 2 H); 7.60 (d, 2 H); 7.32 - 7.36 (m, 1 H); 7.21 - 7.27 (m, 6 H); 7.14 (d, 2 H); 6.85 (t, 1 H); 3.31 - 3.40 (m, 2 H); 3.20- 3.25 (m, 2 H); 1.73-1.84 (m, 2 H); 1.37- 1.40(m,2H); 1.04 - 1.07 (m 2 H). MS (ESI): [M+H]⁺ = 555. |
| **2** | 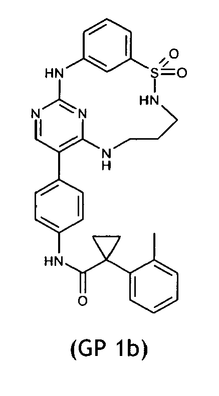 | 1-o-Tolyl-cyclopropane-arboxylic acid [4-(13,13-dioxo-13λ⁶-thia-2,4,8,12,19-pentaaza-tricyclo[12.3.1.1^{3,7}]nonadeca-1(18),3(19),4,6,14,16-hexaen-6-yl)-phenyl]-amide | ¹H-NMR (DMSO, 400 MHz): 9.54 (s, 1 H); 9.40 (s, 1 H); 8.39 (s, 1 H); 7.71-7.75 (m, 1H); 7.70 (s, 1 H); 7.51 (dd, 2 H); 7.32 - 7.40 (m, 2 H); 7.18 - 7.26 (m, 7 H); 6.84 (t, 1 H); 3.32 - 3.38 (m, 2 H); 3.20 - 3.25 (m, 2 H); 2.29 (s, 3 H); 1.74 - 1.82 (m, 2 H); 1.52 - 1.55 (m, 2 H); 1.04 - 1.07(m,2H). MS (ESI): [M+H]⁺ = 555. |
| **3** | 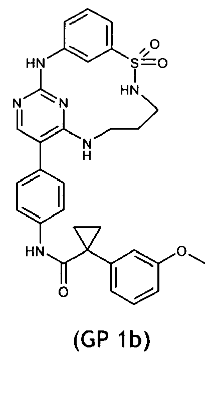 | 1-(3-Methoxy-phenyl)-cyclopropane-carboxylic acid [4-(13,13-dioxo-13λ⁶-thia-2,4,8,12,19-pentaaza-tricyclo[12.3.1.1^{3,7}]nonadeca-1(18),3(19),4,6,14,16-hexaen-6-yl)-phenyl]-amide | ¹H-NMR (DMSO, 300 MHz): 9.54 (s, 1 H); 9.41 (s, 1 H); 9.16 (s, 1 H); 7.70 - 7.74 (m, 2 H); 7.61 (dd, 2 H); 7.34 (t, 1 H); 7.22 - 7.27 (m, 5 H); 6.81 - 6.95 (m, 4 H); 3.73 (s, 3 H); 3.32 - 3.40 (m, 2 H); 3.19 - 3.25 (m, 2 H); 1.74 - 1.85 (m, 2 H); 1.37 - 1.41 (m, 2 H); 1.08- 1.12 (m, 2 H). MS (ESI): [M+H]⁺ = 571. |
| **4** | 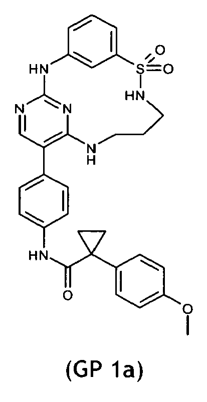 | 1-(4-Methoxy-phenyl)-cyclopropane-carboxylic acid [4-(13,13-dioxo-13λ⁶-thia-2,4,8,12,19-pentaaza-tricyclo[12.3.1.1^{3,7}]-nonadeca-1(18),3(19),4,6,14,16-hexaen-6-yl)-phenyl]-amide | ¹H-NMR (DMSO, 400 MHz): 9.55 (s, 1 H); 9.41 (s, 1 H); 8.93 (s, 1 H); 7.70 - 7.71 (m, 2 H); 7.59 (d, 2 H); 7.22 - 7.36 (m, 7 H); 6.90 (d, 2 H); 6.85 (t, 1 H); 3.72 (s, 3 H); 3.31 - 3.39 (m, 2 H); 3.20 - 3.25 (m, 2 H); 1.73 - 1.83 (m, 2 H); 1.36 - 1.39 (m, 2 H); 1.02- 1.04 (m 2 H). MS (ESI):[M+H]⁺ = 571. |
| **5** | 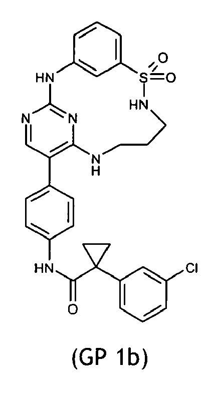 | 1-(3-Chloro-phenyl)-cyclopropane-arboxylic acid [4-(13,13-dioxo-13λ⁶-thia-2,4,8,12,19-pentaaza-tricyclo[12.3.1.1^{3,7}-nadeca-1(18),3(19),4,6,14,16-hexaen-6-yl)-phenyl]-amide | ¹H-NMR (DMSO, 400 MHz): 9.55 (s, 1 H); 9.41 (s, 1 H); 9.38 (s, 1 H); 7.71 - 7.75 (m, 2 H); 7.63 (dd, 2 H); 7.29 - 7.39 (m, 5H); 7.22 - 7.27 (m, 4 H); 6.85 (t, 1 H); 3.32 - 3.38 (m,2 H); 3.20 - 3.25 (m, 2 H); 1.75 - 1.83 (m, 2 H); 1.41 - 1.44 (m, 2 H); 1.13-1.16(m,2H). MS (ESI): [M+H]⁺ = 575. |
| **6** | 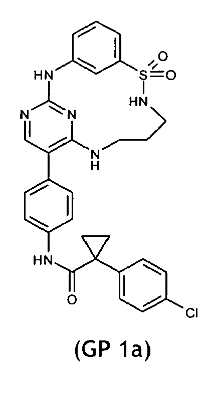 | 1-(4-Chloro-phenyl)-cyclopropane-carboxylic acid [4-(13,13-dioxo-13λ⁶-thia-2,4,8,12,19-pentaaza-tri cyclo[12.3.1.1^{3,7}]-nonadeca-1(18),3(19),4,6,14,16-hexaen-6-yl)-phenyl]-amide | ¹H-NMR (DMSO, 400 MHz): 9.55 (s, 1 H); 9.41 (s, 1 H); 9.26 (s, 1 H); 7.71 - 7.74 (m, 2 H); 7.57 - 7.64 (m, 3 H); 7.32 - 7.41 (m, 4 H); 7.23 - 7.27 (m, 4 H); 6.83 (t, 1 H); 3.31 - 3.38 (m, 2 H); 3.21 - 3.25 (m, 2 H); 1.75 - 1.84 (m, 2 H); 1.42-1.44 (m, 2 H); 1.09-1.11 (m 2 H). MS (ESI): [M+H]⁺ = 575. |
| **7** | 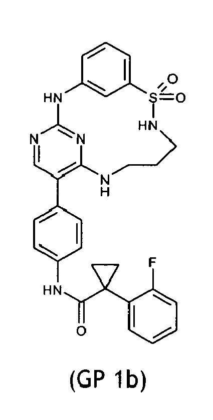 | 1-(2-Fluoro-phenyl)-cyclopropane-carboxylic acid [4-(13,13-dioxo-13λ⁶-thia-2,4,8,12,19-pentaaza-tricyclo[12. 3.1.1^{3,7}]nonadeca-1(18),3(19),4,6,14,16-hexaen-6-yl)-phenyl]-amide | ¹H-NMR (DMSO, 400 MHz): 9.54 (s, 1 H); 9.41 (s, 1 H); 8.83 (s, 1 H); 7.70 - 7.74 (m, 2 H); 7.57 (dd, 2 H); 7.44 (dt, 1 H); 7.32 - 7.38 (m, 2 H); 7.14 - 7.27 (m, 6 H); 6.85 (t, 1 H); 3.31 - 3.38 (m, 2 H); 3.20 - 3.25 (m, 2 H); 1. 75 - 1.83 (m, 2H); 1.52 - 1.55 (m, 2 H); 1.10- 1.13 (m, 2 H). MS (ESI): [M+H]⁺ = 559. |
| **8** | 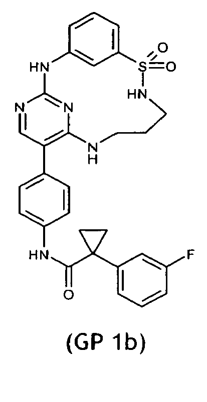 | 1-(3-Fluoro-phenyl)-cyclopropane-carboxylic acid [4-(13, 13-dioxo-13λ⁶-thia-2,4,8,12,19-pentaaza-tricyclo[12.3.1.1^{3,7}]nonadeca-1(18),3(19),4,6,14,16-hexaen-6-yl)-phenyl]-amide | ¹H-NMR (DMSO, 400 MHz): 9.55 (s, 1 H); 9.41 (s, 1 H); 9.35 (s, 1 H); 7.70 - 7.74 (m, 2 H); 7.63 (dd, 2 H); 7.32 - 7.39 (m, 2 H); 7.05 - 7.27 (m, 7 H); 6.85 (t, 1 H); 3.31 - 3.40 (m, 2 H); 3.20 - 3.27 (m, 2 H); 1.75 - 1.83 (m, 2 H);1.41 - 1.44(m,2H); 1.16 - 1.16 (m, 2 H). MS(ESI): [M+H]⁺ = 559. |
| **9** | 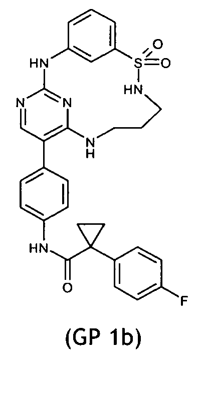 | 1-(4-Fluoro-phenyl)-cyclopropane-carboxylic acid [4-(13,13-dioxo-13λ⁶-thia-2,4,8,12,19-pentaaza-tricyclo[12.3.1.1^{3.7}]nonadeca-1(18),3(19),4,6,14,16-hexaen-6-yl)-phenyl]-amide | ¹H-NMR (DMSO, 300 MHz): 9.54 (s, 1 H); 9.41 (s, 1 H); 9.14 (s, 1 H); 7.70 - 7.74 (m, 2 H); 7.60 (dd, 2 H); 7.32 - 7.44 (m, 3 H); 7.21 - 7.27 (m, 4 H); 7.12 - 7.18 (m, 2 H); 6.84 (t, 1 H); 3.32 - 3.38 (m, 2 H); 3.20 - 3.25 (m, 2 H); 1.74 - 1.84(m, 2 H); 1.40-1.43(m,2H); 1.06 - 1.10 (m, 2 H). MS (ESI): [M+H]⁺ = 559. |
| **10** | 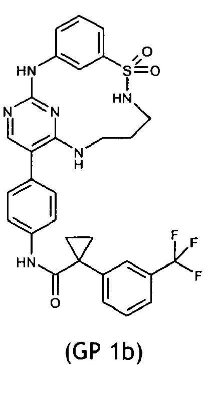 | 1-(3-Trifluoromethyl-phenyl)-cyclopropanecarbox ylic acid [4-(13,13-dioxo-13λ⁶-thia-2,4,8,12,19-pentaaza-tricyclo[12.3.1.1^{3,7}]nonadeca-1(18),3(19),4,6,14,16-hexaen-6-yl)-phenyl]-amide | ¹H-NMR (DMSO, 300 MHz): 9.55 (s, 1 H); 9.45 (s, 1 H); 9.41 (s, 1 H); 7.69 - 7.75 (m, 2 H); 7.51 - 7.68 (m, 6 H); 7.34 (t, 1 H); 7.21 - 7.27 (m, 4 H); 6.85 (t, 1 H); 3.32-3.40 (m, 2 H); 3.20 - 3.25 (m, 2 H); 1.74 - 1.85 (m, 2 H); 1.46 - 1.49 (m, 2H); 1.17 - 1.21 (m, 2 H). MS (ESI): [M+H]⁺ = 609. |
| **11** | 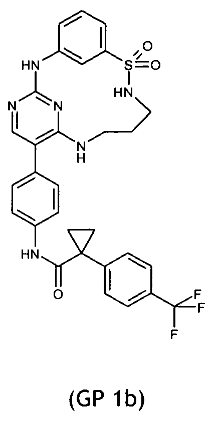 | 1-(4-Trifluoromethyl-phenyl)-cyclopropane-carboxylic acid [4-(13, 13-dioxo-13λ⁶-thia-2,4,8,12,19-pentaaza-tricyclo[12.3.1.1.^{3,7}]nonadeca-1(18),3(19),4,6,14,16-hexaen-6-yl)-phenyl]-amide | ¹H-NMR (DMSO, 400 MHz): 9.55 (s, 1 H); 9.47 (s, 1 H); 9.41 (s, 1 H); 7.72 (s, 1 H); 7.71 (t, 1 H); 7.68 (d, 2 H); 7.63 (dd, 2 H); 7.54 (d, 2 H); 7.30-7.37 (m, 2 H); 7.23 - 7.26 (m, 3 H); 6.85 (t, 1 H); 3.32 - 3.39 (m, 2 H); 3.21 - 3.25 (m, 2 H); 1.75 - 1.83 (m, 2 H); 1.47 - 1.50 (m, 2 H); 1.17 - 1.20 (m,2H). MS (ESI): [M+H]⁺ = 609. |
| **12** | 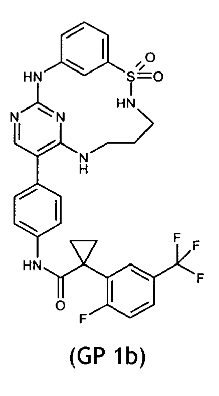 | 1-(2-Fluoro-5-trifluoromethyl-phenyl)-cyclopropane-carboxylic acid [4-(13,13-dioxo-13λ⁶-thia-2,4,8,12,19-pentaaza-tricyclo[12.3.1.1^{3,7}]-nonadeca-1(18),3(19),4,6,14,16-hexaen-6-yl)-phenyl]-amide | ¹H-NMR (DMSO, 400 MHz): 9.55 (s, 1 H); 9.41 (s, 1 H); 9.05 (s, 1 H); 7.71 - 7.80 (m, 4 H); 7.54 - 7.58 (m, 2 H); 7.21 - 7.44 (m, 6 H); 6.86 (t, 1 H); 3.32 - 3.39 (m, 2 H); 3.20 - 3.27 (m, 2 H); 1.74 - 1.84 (m, 2 H); 1.56 - 1.58 (m, 2 H); 1.20 - 1.24 (m, 2 H). MS (ESI): [M+H]⁺ = 627. |
| **13** | 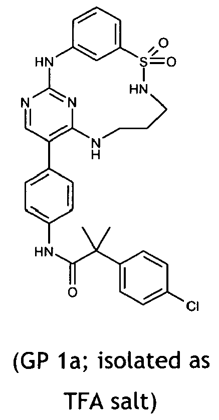 | 2-(4-Chloro-phenyl)-N-[4-(13,13-dioxo-13λ⁶-thia-2,4,8,12,19-pentaaza-tricyclo[12.3.1.1^{3,7}]-nonadeca-1(18),3(19),4,6,14,16-hexaen-6-yl)-phenyl]-isobutyramide | ¹H-NMR (DMSO, 400 MHz): 10.39 (s, 1 H); 9.26 (s, 1 H); 9.10 (s, 1 H); 7.97 (br. s, 1 H); 7.70 - 7.79 (m, 4 H); 7.24 - 7.50 (m, 9 H); 3.30 - 3.40 (m, 2 H); 3.20 - 3.28 (m, 2 H); 1.72 - 1.82 (m, 2 H); 1.53 (s, 6 H). MS (ESI):[M+H]⁺ = 576 |

The following Example Compounds can be obtained using the methods described before or by standard procedures known to those skilled on the art:

| | | | |
|---|---|---|---|
| 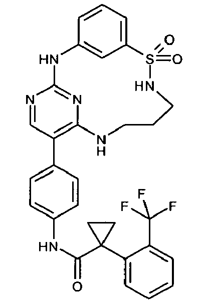 | 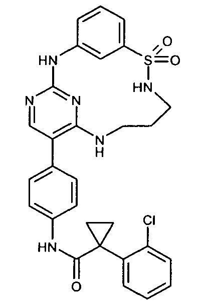 | 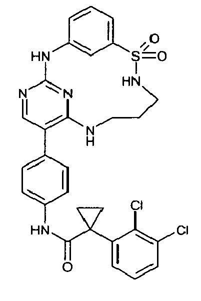 | 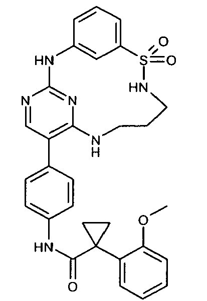 |
| **Example 14** | **Example 15** | **Example 16** | **Example 17** |
| 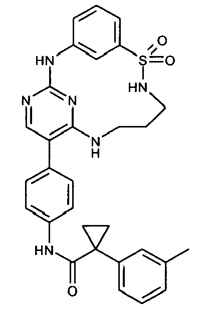 | 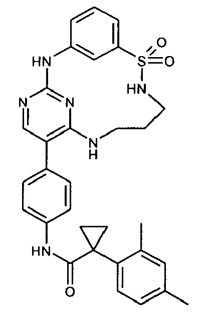 | 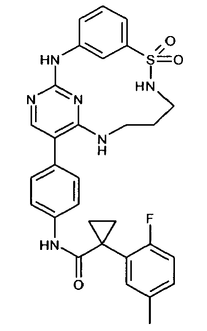 | 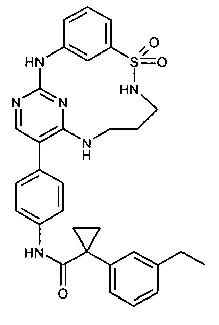 |
| **Example 18** | **Example 19** | **Example 20** | **Example 21** |
| 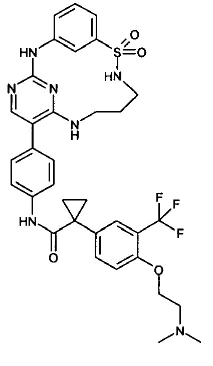 | 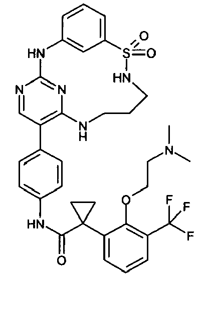 | 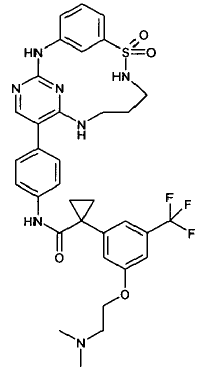 | 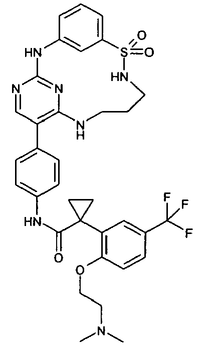 |
| **Example 22** | **Example 23** | **Example 24** | **Example 25** |
| 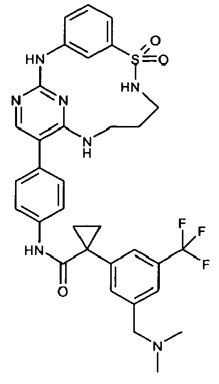 | 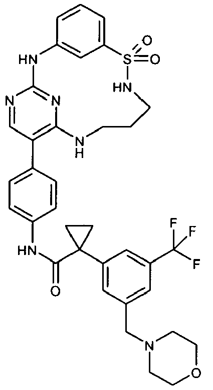 | 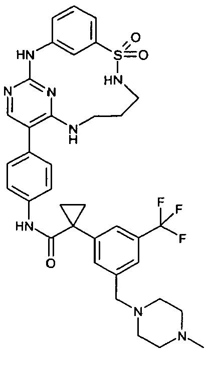 | 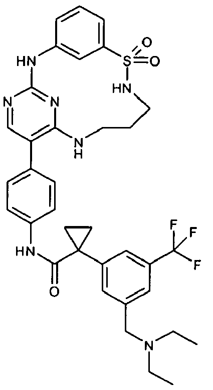 |
| **Example 26** | **Example 27** | **Example 28** | **Example 29** |
| 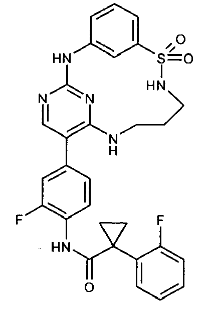 | 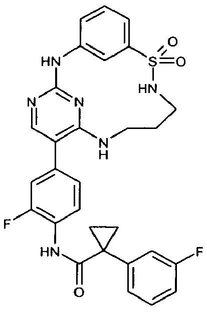 | 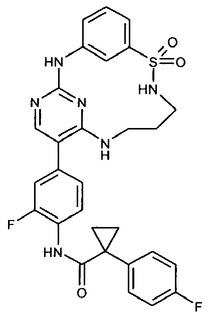 | 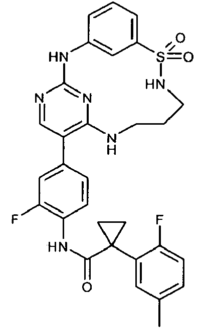 |
| **Example 30** | **Example 31** | **Example 32** | **Example 33** |
| 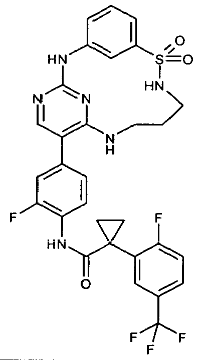 | 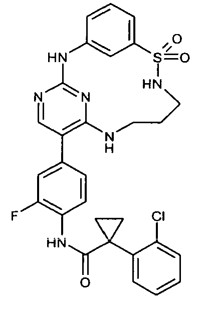 | 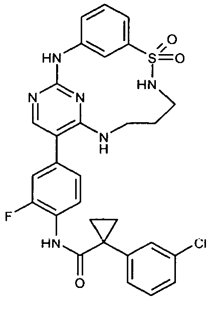 | 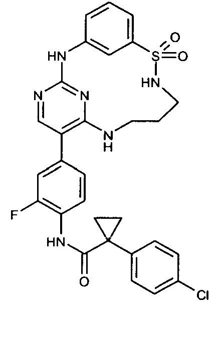 |
| **Example 34** | **Example 35** | **Example 36** | **Example 37** |
| 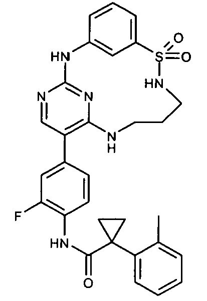 | 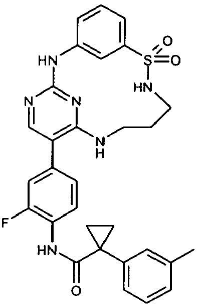 | 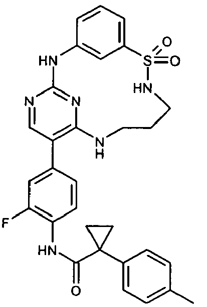 | 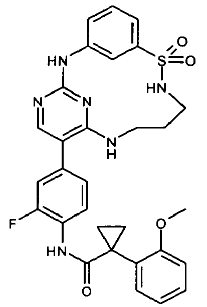 |
| **Example 38** | **Example 39** | **Example 40** | **Example 41** |
| 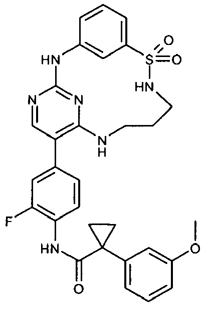 | 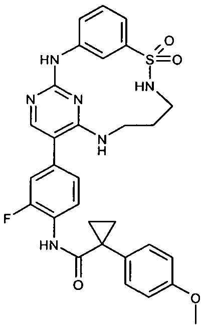 | 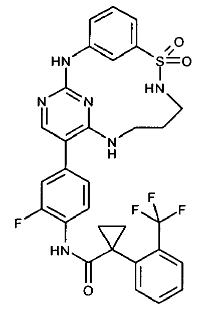 | 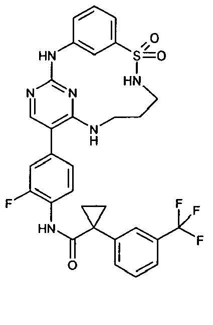 |
| **Example 42** | **Example 43** | **Example 44** | **Example 45** |
| 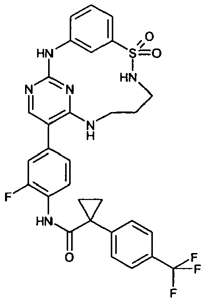 | 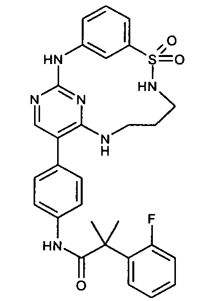 | 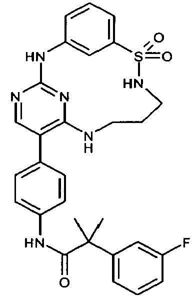 | 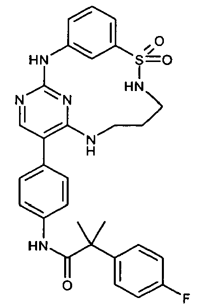 |
| **Example 46** | **Example 47** | **Example 48** | **Example 49** |
| 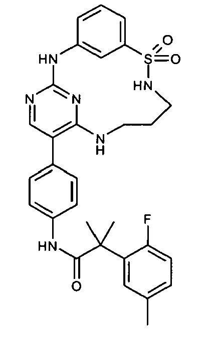 | 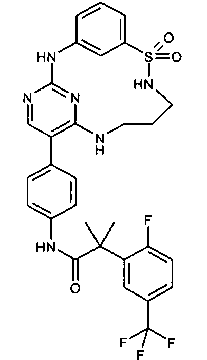 | 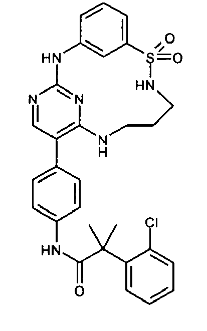 | 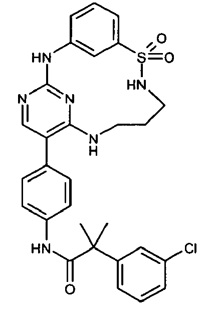 |
| **Example 50** | **Example 51** | **Example 52** | **Example 53** |
| 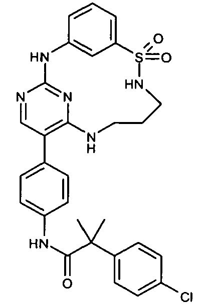 | 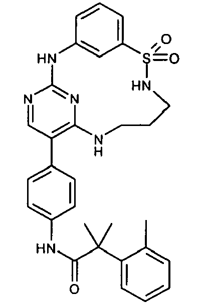 | 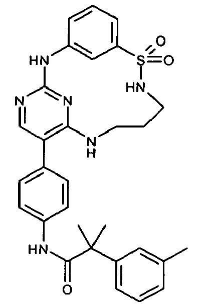 | 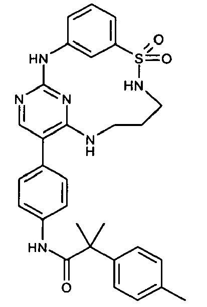 |
| **Example 54** | **Example 55** | **Example 56** | **Example 57** |
| 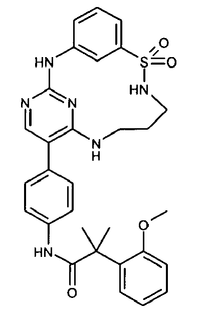 | 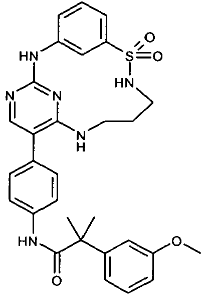 | 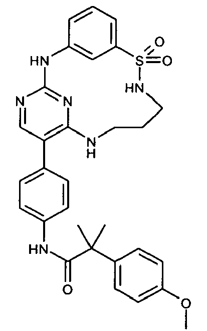 | 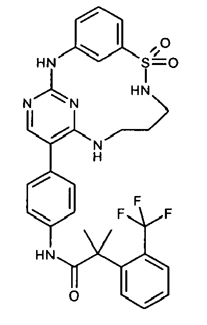 |
| **Example 58** | **Example 59** | **Example 60** | **Example 61** |
| 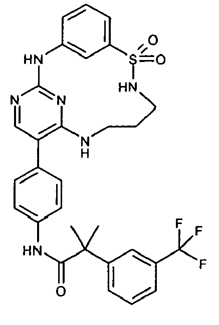 | 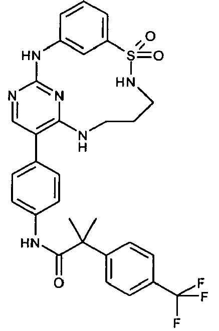 | 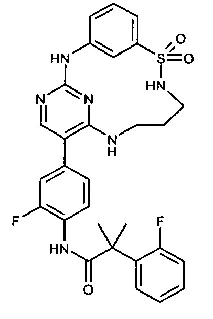 | 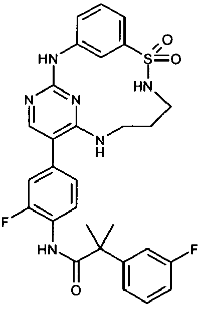 |
| **Example 62** | **Example 63** | **Example 64** | **Example 65** |
| 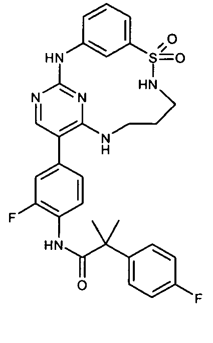 | 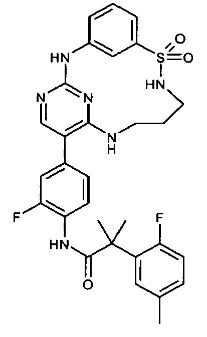 | 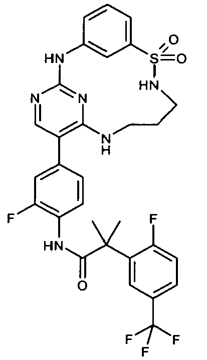 | 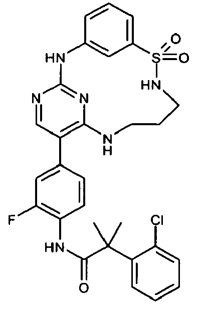 |
| **Example 66** | **Example 67** | **Example 68** | **Example 69** |
| 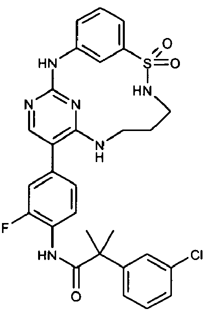 | 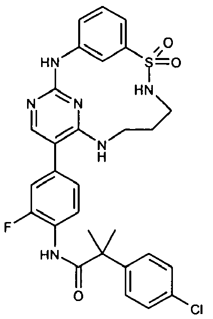 | 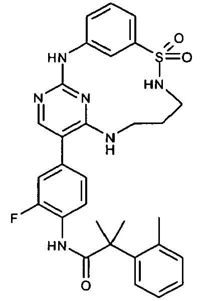 | 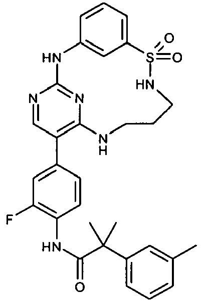 |
| **Example 70** | **Example 71** | **Example 72** | **Example 73** |
| 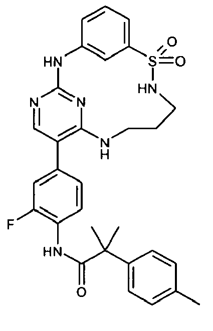 | 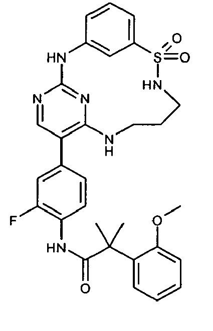 | 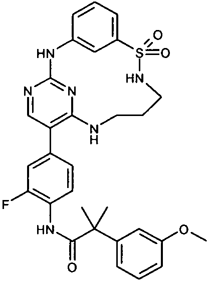 | 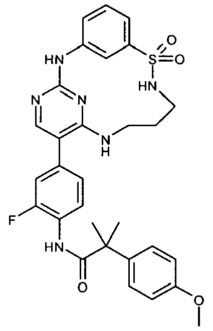 |
| **Example 74** | **Example 75** | **Example 76** | **Example 77** |
| 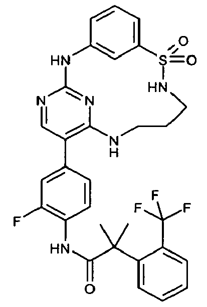 | 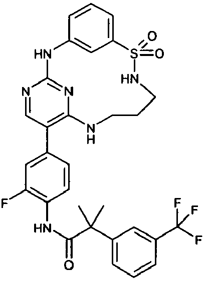 | 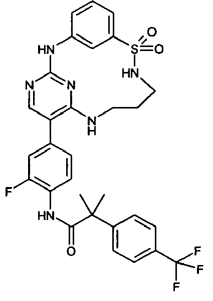 | 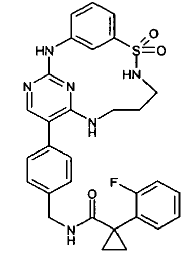 |
| **Example 78** | **Example 79** | **Example 80** | **Example 81** |
| 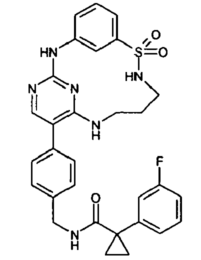 | 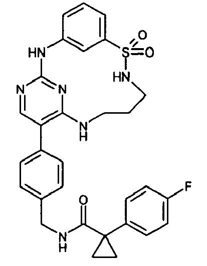 | 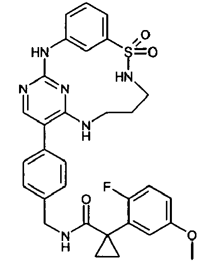 | 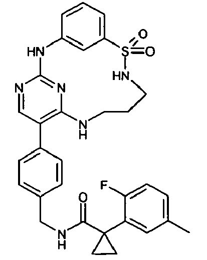 |
| **Example 82** | **Example 83** | **Example 84** | **Example 85** |
| 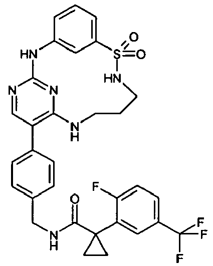 | 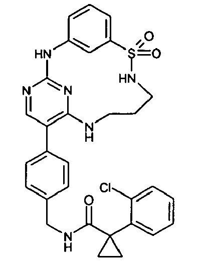 | 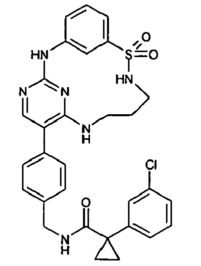 | 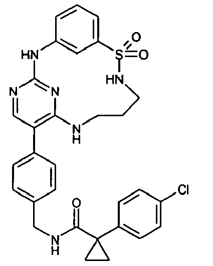 |
| **Example 86** | **Example 87** | **Example 88** | **Example 89** |
| 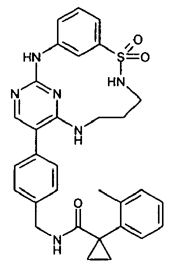 | 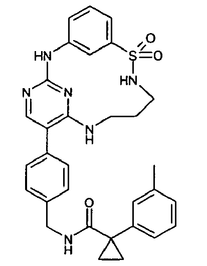 | 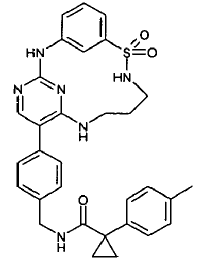 | 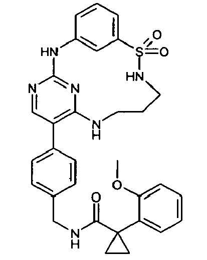 |
| **Example 90** | **Example 91** | **Example 92** | **Example 93** |
| 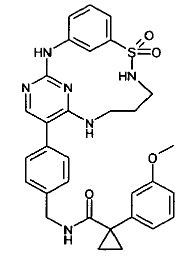 | 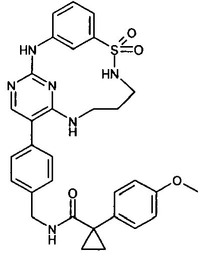 | 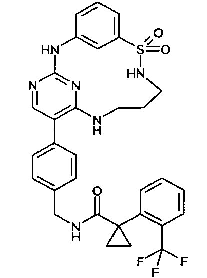 | 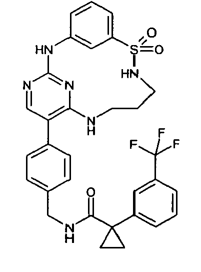 |
| **Example 94** | **Example 95** | **Example 95** | **Example 96** |
| 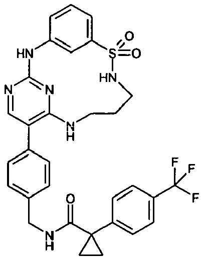 | 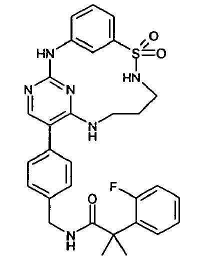 | 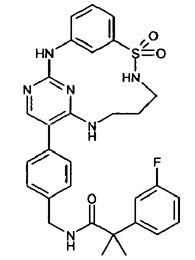 | 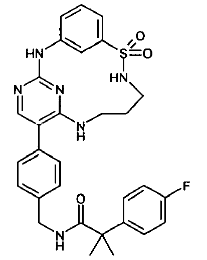 |
| **Example 97** | **Example 98** | **Example 99** | **Example 100** |
| 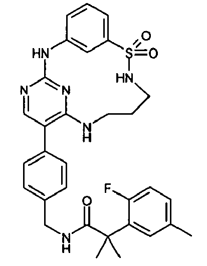 | 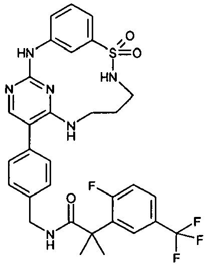 | 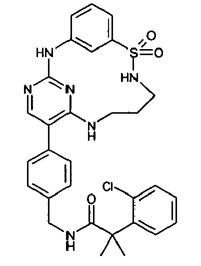 | 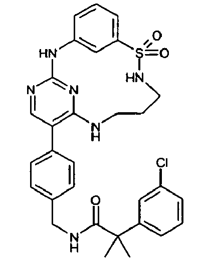 |
| **Example 101** | **Example 102** | **Example 103** | **Example 104** |
| 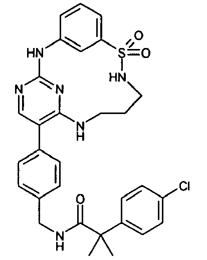 | 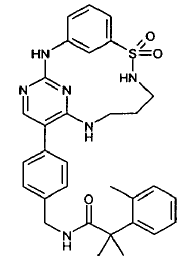 | 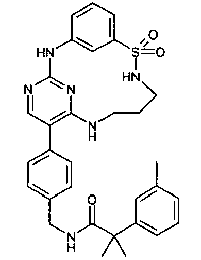 | 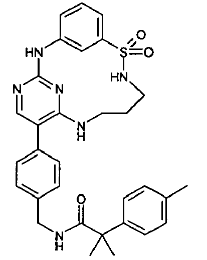 |
| **Example 105** | **Example 106** | **Example 107** | **Example 108** |
| 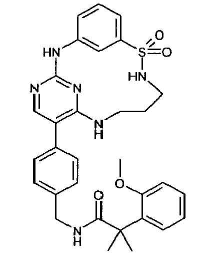 | 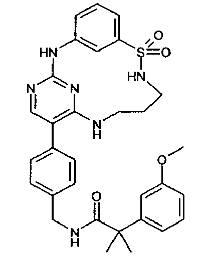 | 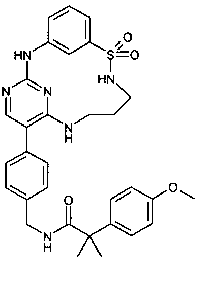 | 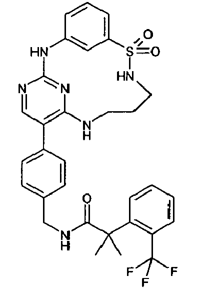 |
| **Example 109** | **Example 110** | **Example 111** | **Example 112** |
| 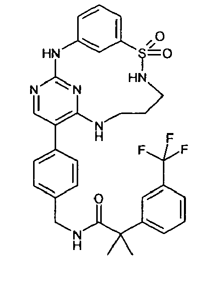 | 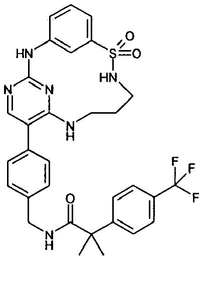 | 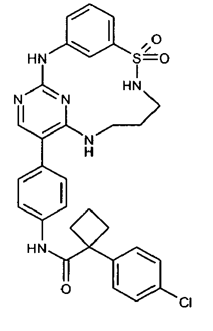 | 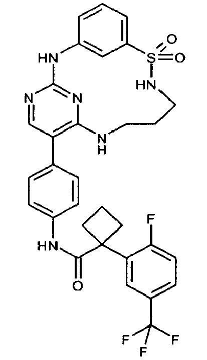 |
| **Example 113** | **Example 114** | **Example 115** | **Example 116** |
| 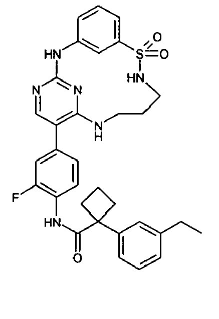 | 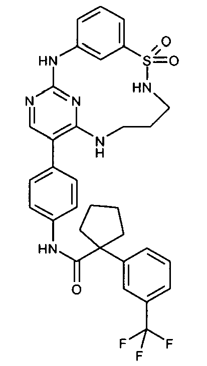 | 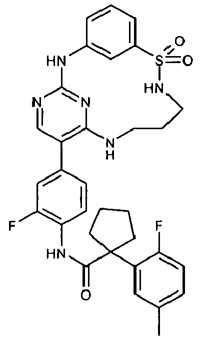 | 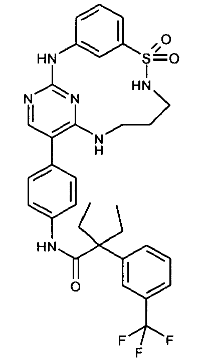 |
| **Example 117** | **Example 118** | **Example 119** | **Example 120** |
| 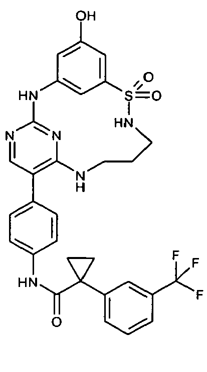 | 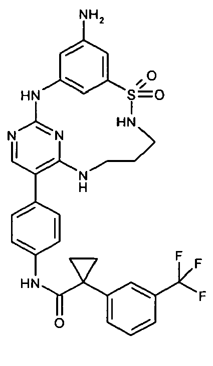 | 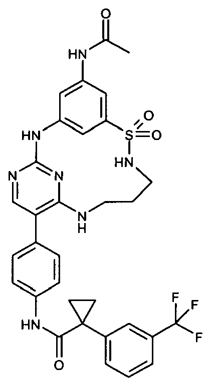 | 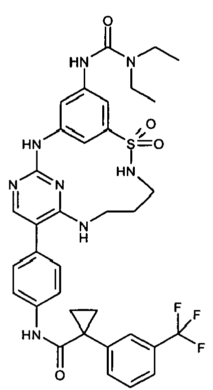 |
| **Example 121** | **Example 122** | **Example 123** | **Example 124** |
| 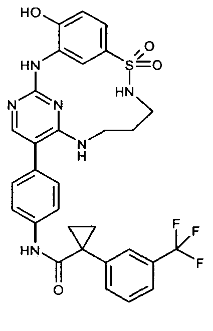 | 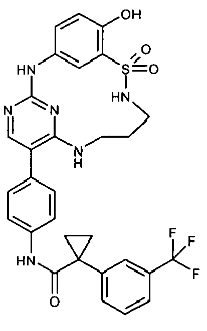 | 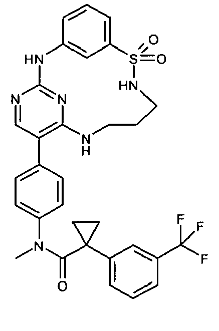 | 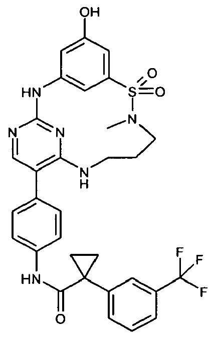 |
| **Example 125** | **Example 126** | **Example 127** | **Example 128** |

### BIOLOGICAL DATA

### Assay 1: Tie2 ELISA Assay

Cellular activity of compounds of the present invention as inhibitors of Tie2 kinase activity was measured employing a Tie2 ELISA assay as described in the following paragraphs. Herein CHO cell-cultures, which are stably transfected by known techniques with Tie2 using DHFR deficiency as selection marker, are stimulated by angiopoietin-2. The specific autophosphorylation of Tie2 receptors is quantified with a sandwich-ELISA using anti-Tie2 antibodies for catch and anti-phosphotyrosine antibodies coupled to HRP for detection.

### Materials:

96well tissue culture plate, sterile, Greiner
96well FluoroNunc plate MaxiSorp Surface C, Nunc
96well plate polypropylene for compound dilution in DMSO
CHO Tie2/DHFR (transfected cells)
PBS-; PBS++, DMSO
MEM alpha Medium with Glutamax-I without Ribonucleosides and
   Deoxyribonucleosides (Gibco #32561-029)
   with 10% FCS after dialysis! and 1% PenStrep
Lysis buffer: 1 Tablet "Complete" protease inhibitor
   1 cap Vanadate (1 mL > 40 mg/mL; working solution 2 mM)
   ad 50 mL with Duschl-Puffer
   pH 7.6
Anti-Tie2-antibody 1 : 425 in Coating Buffer pH 9.6
   Stock solution: 1.275 mg/mL > working.: 3 µg/mL
PBST: 2 bottles PBS(10x) + 10ml Tween, fill up with VE-water
RotiBlock 1 : 10 in VE-water
Anti-Phosphotyrosine HRP-Conjugated 1 : 10000 in 3% TopBlock
   3% TopBlock in PBST
BM Chemiluminescence ELISA Substrate (POD)
   solution B 1 : 100 solution A
SF9 cell culture medium
Ang2-Fc in SF9 cell culture medium

### Cell experiment:

Dispense 5 x 10⁴ cells / well / 98 µL in 96well tissue culture plate
Incubate at 37 °C / 5% CO₂
After 24 h add compounds according to desired concentrations
Add also to control and stimulated values without compounds 2 µL DMSO
And mix for a few min at room temperature
Add 100 µL Ang2-Fc to all wells except control, which receives insect medium
Incubate 20 min at 37 °C.
Wash 3x with PBS++
Add 100 µl Lysis buffer / well and shake a couple of min at room temperature
Store lysates at 20 °C before utilizing for the ELISA

### Performance of sandwich-ELISA

Coat 96well FluoroNunc Plate MaxiSorp Surface C with anti-Tie2 mAb
1 : 425 in Coating buffer pH 9.6; 100 µL / well overnight at 4 °C
Wash 2x with PBST
Block plates with 250 µL / well RotiBlock 1 : 10 in VE-water
Incubate for 2 h at room temperature or overnight at 4 °C shaking
Wash 2x in PBST
Add thawed lysates to wells and incubate overnight shaking at 4 °C
Wash 2x with PBST
Add 100 µL / well anti-Phosphotyrosine HRP-Conjugated 1 : 10000 in 3%
TopBlock (3% TopBlock in PBST) and incubate overnight under shaking
Wash 6x with PBST
Add 100 µL / well BM Chemiluminescence ELISA Substrate (POD)
solutions 1 und 2 (1 : 100)
Determine luminescence with the LumiCount.

### Assay 2: Tie-2-Kinase HTRF-Assay without kinase preactivation

Tie2-inhibitory activity of compounds of the present invention was quantified employing two Tie2 HTRF assay as described in the following paragraphs.

A recombinant fusion protein of GST and the intracellular domains of Tie-2, expressed in insect cells (Hi-5) and purified by Glutathion-Sepharose affinity chromatography was used as kinase. Alternatively, commercially available GST-Tie2-fusion protein (Upstate Biotechnology, Dundee, Scotland) can be used As substrate for the kinase reaction the biotinylated peptide biotin-Ahx-EPKDDAYPLYSDFG (C-terminus in amid form) was used which can be purchased e.g. from the company Biosynthan GmbH (Berlin-Buch, Germany). Detection of phosphorylated product is achieved specifically by a trimeric detection complex consisting of the phosphorylated substrate, streptavidin-XLent (SA-XLent) which binds to biotin, and Europium Cryptate-labeled anti-phosphotyrosine antibody PT66 which binds to phosphorylated tyrosine.

Tie-2 (3.5 ng/measurement point) was incubated for 60 min at 22°C in the presence of 10 µM adenosine-tri-phosphate (ATP) and 1 µM substrate peptide (biotin-Ahx-EPKDDAYPLYSDFG-NH₂) with different concentrations of test compounds (0 µM and concentrations in the range 0.001 - 20 µM) in 5 µl assay buffer [50 mM Hepes/NaOH pH 7, 10 mM MgCl₂, 0.5 mM MnCl₂, 1.0 mM dithiothreitol, 0.01% NP40, protease inhibitor mixture ("Complete w/o EDTA" from Roche, 1 tablet per 2.5 ml), 1 % (v/v) dimethylsulfoxide]. The reaction was stopped by the addition of 5 µl of an aqueous buffer (25 mM Hepes/NaOH pH 7.5, 0.28 % (w/v) bovine serum albumin) containing EDTA (90 mM) and the HTRF (Homogeneous Time Resolved Fluorescence) detection reagents streptavidine-XLent (0.2 µM, from Cis Biointernational, Marcoule, France) and PT66-Eu-Chelate (0.3 ng/µl; a europium-chelate labelled anti-phospho-tyrosine antibody from Perkin Elmer).

The resulting mixture was incubated 1 h at 22°C to allow the binding of the biotinylated phosphorylated peptide to the streptavidine-XLent and the PT66-Eu-Chelate. Subsequently the amount of phosphorylated substrate peptide was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate peptide. The data were normalised (enzyme reaction without inhibitor = 0 % inhibition, all other assay components but no enzyme = 100 % inhibition) and IC₅₀ values were calculated by a 4 parameter fit using an inhouse software.

### Assay 3: Tie-2-Kinase HTRF-Assay with kinase preactivation

A recombinant fusion protein of GST and the intracellular domains of Tie-2, expressed in insect cells (Hi-5) and purified by Glutathion-Sepharose affinity chromatography was used as kinase. As substrate for the kinase reaction the biotinylated peptide biotin-Ahx-EPKDDAYPLYSDFG (C-terminus in amid form) was used which can be purchased e.g. from the company Biosynthan GmbH (Berlin-Buch, Germany).

For activation, Tie-2 was incubated at a conc. 12.5 ng/µl of for 20 min at 22°C in the presence of 250 µM adenosine-tri-phosphate (ATP) in assay buffer [50 mM Hepes/NaOH pH 7, 10 mM MgCl₂, 0.5 mM MnCl₂, 1.0 mM dithiothreitol, 0.01% NP40, protease inhibitor mixture ("Complete w/o EDTA" from Roche, 1 tablet per 2.5 ml)].

For the subsequent kinase reaction, the preactivated Tie-2 (0.5 ng/measurement point) was incubated for 20 min at 22°C in the presence of 10 µM adenosine-tri-phosphate (ATP) and 1 µM substrate peptide (biotin-Ahx-EPKDDAYPLYSDFG-NH₂) with different concentrations of test compounds (0 µM and concentrations in the range 0.001 - 20 µM) in 5 µl assay buffer [50 mM Hepes/NaOH pH 7, 10 mM MgCl₂, 0.5 mM MnCl₂, 0.1 mM sodium ortho-vanadate, 1.0 mM dithiothreitol, 0.01% NP40, protease inhibitor mixture ("Complete w/o EDTA" from Roche, 1 tablet per 2.5 ml), 1 % (v/v) dimethylsulfoxide]. The reaction was stopped by the addition of 5 µl of an aqueous buffer (25 mM Hepes/NaOH pH 7.5, 0.28% (w/v) bovine serum albumin) containing EDTA (90 mM) and the HTRF (Homogeneous Time Resolved Fluorescence) detection reagents streptavidine-XLent (0.2 µM, from Cis Biointernational, Marcoule, France) and PT66-Eu-Chelate (0.3 ng/µl; a europium-chelate labelled anti-phospho-tyrosine antibody from Perkin Elmer).

The resulting mixture was incubated 1 h at 22°C to allow the binding of the biotinylated phosphorylated peptide to the streptavidine-XLent and the PT66-Eu-Chelate. Subsequently the amount of phosphorylated substrate peptide was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate peptide. The data were normalised (enzyme reaction without inhibitor = 0 % inhibition, all other assay components but no enzyme = 100 % inhibition) and IC₅₀ values were calculated by a 4 parameter fit using an inhouse software.

### Assay 4: CDK2 HTRF Assay

CDK2/CycE -inhibitory activity of compounds of the present invention was quantified employing the CDK2/CycE HTRF assay as described in the following paragraphs.

Recombinant fusion proteins of GST and human CDK2 and of GST and human CycE, expressed in insect cells (Sf9) and purified by Glutathion-Sepharose affinity chromatography, were purchase from ProQinase GmbH (Freiburg, Germany). As substrate for the kinase reaction biotinylated peptide biotin-Ttds-YISPLKSPYKISEG (C-terminus in amid form) was used which can be purchased e.g. form the company JERINI peptide technologies (Berlin, Germany).
CDK2/CycE was incubated for 60 min at 22°C in the presence of different concentrations of test compounds in 5 µl assay buffer [50 mM Tris/HCl pH 8.0, 10 mM MgCl2, 1.0 mM dithiothreitol, 0.1 mM sodium ortho-vanadate, 10 µM adenosine-tri-phosphate (ATP), 0.75 µM substrate, 0.01% (v/v) Nonidet-P40 (Sigma), 1 % (v/v) dimethylsulfoxide]. The concentration of CDK2/CycE was adjusted depending of the activity of the enzyme lot and was chosen appropriate to have the assay in the linear range, typical concentrations were in the range of 1 ng/ml. The reaction was stopped by the addition of 5 µl of a solution of HTRF detection reagents (0.2 µM streptavidine-XLent and 3.4 nM Phospho-(Ser) CDKs Substrate Antibody [product #2324B, Cell Signalling Technology, Danvers, MA, USA} and 4 nM Prot-A-EuK [Protein A labeled with Europium Cryptate from Cis biointernational, France, product no. 61 PRAKLB]) in an aqueous EDTA-solution (100 mM EDTA, 800 mM KF, 0.2 % (w/v) bovine serum albumin in 100 mM HEPES/NaOH pH 7.0).

### Assay 5: Aurora C HTRF Assay

Aurora-C inhibitory activity of compounds of the present invention was quantified employing the Aurora-C HTRF assay as described in the following paragraphs.

Recombinant fusion protein of GST and human Aurora-C was expressed in transiently transfected HEK293 cells (Sf9) and purified by Glutathion-Sepharose affinity chromatography. As substrate for the kinase reaction biotinylated peptide biotin-Ttds-FMRLRRLSTKYRT (C-terminus in amid form) was used which can be purchased e.g. form the company JERINI peptide technologies (Berlin, Germany). Aurora-C was incubated for 60 min at 22°C in the presence of different concentrations of test compounds in 5 µl assay buffer [25 mM Hepes/NaOH pH 7.4, 0.5 mM MnCl2, 2.0 mM dithiothreitol, 0.1 mM sodium ortho-vanadate, 10 µM adenosine-tri-phosphate (ATP), 0.5 µM/ml substrate, 0.01% (v/v) TritonX-100 (Sigma), 0.05 % (w/v) bovine serum albumin, 1 % (v/v) dimethylsulfoxide]. The concentration of Aurora-C was adjusted depending of the activity of the enzyme lot and was chosen appropriate to have the assay in the linear range, typical concentrations were in the range of 0.3 nM. The reaction was stopped by the addition of 5 µl of a solution of HTRF detection reagents (0.2 µM streptavidine-XLent and and 1.4 nM Anti-Phospho-(Ser/Thr) Akt substrate-Cryptate (Cis biointernational, France, product no. 61 P02KAE), a labeled Phospho-(Ser/Thr) Akt substrate antibody [product #9611 B, Cell Signalling Technology, Danvers, MA, USA] labeled with Europium Cryptate, in an aqueous EDTA-solution (40 mM EDTA, 400 mM KF, 0.05 % (w/v) bovine serum albumin in 25 mM HEPES/NaOH pH 7.0).

The resulting mixture was incubated 1 h at 22°C to allow the binding of the biotinylated phosphorylated peptide to the streptavidine-XLent and the Anti-Phospho-(Ser/Thr) Akt substrate-Cryptate. Subsequently the amount of phosphorylated substrate was evaluated by measurement of the resonance energy transfer from the Prot-A-EuK to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition) and IC50 values were calculated by a 4 parameter fit using an inhouse software.

Compounds of the present invention were found to possess high enzymatic and cellular activity as inhibitors of Tie2 kinase. Compounds of the present invention inhibit Tie2 kinase activity and cellular Tie2 autophosphorylation with IC₅₀ values below 0.5 µM, more preferred compounds inhibit Tie2 autophosphorylation with IC₅₀ values below 0.1 µM. Surprisingly, it was found that compounds of the present invention were found to possess a selectivity profile, which is highly advantageous for Tie2 inhibitors to be used as purely antiangiogenic agents, as they are more potently inhibiting Tie2 activity than inhibiting the cell cycle kinases CDK2 and Aurora C. The selectivity of Tie2-inhibitors of the present invention vs. CDK2 inhibition is greater than 20fold (as expressed by IC₅₀ assay 4 / IC₅₀ assay 2). The selectivity of Tie2-inhibitors of the present invention vs. Aurora C inhibition is greater than 20fold (as expressed by IC₅₀ assay 5 / IC₅ₒ assay 2).

Selected data are given in the following table. The IC₅₀ values were converted to pIC₅₀ values, i.e. -log IC₅₀ in molar concentration.

| **Example No.** | **Tie 2 activity** **(assay 1)** | **Tie 2 activity** **(assay 2)** | **Selectivity vs. CDK2** | **Selectivity vs. Aurora C** |
|---|---|---|---|---|
| 1 | ++ | ++ | > 20fold | > 20fold |
| 2 | ++ | ++ | > 20fold | > 20fold |
| 3 | ++ | ++ | > 20fold | > 20fold |
| 4 | ++ | ++ | > 20fold | > 20fold |
| 5 | ++ | ++ | > 20fold | > 20fold |
| 6 | ++ | ++ | > 20fold | > 20fold |
| 7 | ++ | ++ | > 20fold | > 20fold |
| 8 | ++ | ++ | > 20fold | > 20fold |
| 9 | ++ | ++ | > 20fold | > 20fold |
| 10 | ++ | + | > 20fold | > 20fold |
| 11 | ++ | ++ | > 20fold | > 20fold |
| 12 | ++ | ++ | > 20fold | > 20fold |
| 13 | + | ++ | > 20fold | > 20fold |

| | | | | |
|---|---|---|---|---|
| + stands for PIC₅₀ 6.0 - 7.0 ++ stands for PIC₅₀ > 7.0 Selectivity vs. CDK2: IC₅₀ assay 4 / IC₅₀ assay 2 Selectivity vs. Aurora C: IC₅₀ assay 5 / IC₅₀ assay 2 | | | | |

## Claims

1. A compound of general formula (I) : 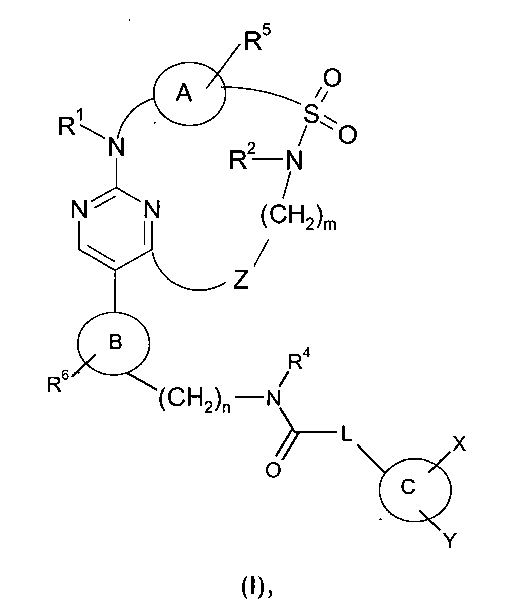 wherein
A is phenylene or C₆-heteroarylene;
B, C are, independently from each other, arylene or heteroarylene ;
Z is selected from the group comprising, preferably consisting of, O, S, NR³ and CHR³ ;
R¹, R², R³ and R⁴ independently from each other are selected from the group comprising, preferably consisting of, hydrogen and C₁-C₆-alkyl, wherein C₁-C₆-alkyl is unsubstituted or substituted one or more times with -OR⁷ or -NR⁷R⁸;
R⁵ is selected from the group comprising, preferably consisting of, hydrogen, halogen, nitro, cyano, C₁-C₆-alkyl, -(CH₂)ₚOR^{7a}, - (CH₂)ₚNR^{7a}R^{8a}, -(CH₂)ₚC(O)R^{7a}, -(CH₂)ₚNHC(O)R^{7a}, - (CH₂)ₚNHC(O)NR^{7a}R^{8a}, -(CH₂)ₚNHS(O)₂R^{7a}, and -(CH₂)ₚC(O)NR^{7a}R^{8a} ;
R⁶ is selected from the group comprising, preferably consisting of, hydrogen, halogen, nitro, cyano, C₁-C₆-alkyl, -OR^{7b}, -NR^{7b}R^{8b}, C₁-C₆-haloalkyl, C₁-C₆-haloalkoxy, C₁-C₆-alkylthio and C₁-C₆-alkylcarbonyl ;
R⁷ , R^{7a} , R^{7b}, R^{7c}, R^{7d}, R^{7e} , R^{7f}, R⁸, R^{8a} , R^{8b} , R^{8c}, R^{8d}, R^{8e}, R^{8f} independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7c}, -NR^{7c}R^{8c}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
R⁷ and R⁸, R^{7a} and R^{8a} , R^{7b} and R^{8b} , R^{7c} and R^{8c}, R^{7e} and R^{8e} , and R^{7f} and R^{8f} independently from each other, together with the nitrogen atom to which they are attached in groups -NR⁷R⁸, -NR^{7a}R^{8a}, -NR^{7b}R^{8b}, -NR^{7c}R^{8c} , -NR^{7e}R^{8e}, and -NR^{7f}R^{8f} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a - C(O)-, -S(O)- , and -S(O)₂- group, and can optionally contain one or more double bonds ;
R⁹, R^{9a}, independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
R⁹ and R^{9a}, together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
L is selected from the group comprising, preferably consisting of C₁-C₆-alkylene or C₃-C₁₀-cycloalkylene ;
X is selected from the group comprising, preferably consisting of, halogen, nitro, cyano, -(CH₂)ᵣOR^{7e}, -(CH₂)ᵣNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7e}, -(CH₂)ᵣC(O)NR^{7e}R^{8e} and - (CH₂)ᵣS(O)₂NR^{7e}R^{8e} ;
Y is selected from the group comprising, preferably consisting of, hydrogen, halogen, nitro, cyano, -(CH₂)ₛOR^{7f}, -(CH₂)ₛNR^{7f}R^{8f}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7f}, -(CH₂)ₛC(O)NR^{7f}R^{8f} and - (CH₂)ₛS(O)₂NR^{8f} ;
m represents an integer of 3, 4, 5, or 6 ;
n, p, q, q', r, s independently from each other represent an integer of 0, 1, or 2 .

2. The compound of general formula (I) according to claim 1, wherein :
A is meta-phenylene ;
B, C are, independently from each other, arylene or heteroarylene ;
Z is N_{R}³ ;
R¹, R², R³, and R⁵ are hydrogen ;
R⁴ is hydrogen or C₁-C₆-alkyl ;
R⁶ is selected from the group comprising, preferably consisting of, hydrogen, halogen, nitro, cyano, C₁-C₆-alkyl, -OR^{7b}, -NR^{7b}R^{8b}, C₁-C₆-haloalkyl, C₁-C₆-haloalkoxy, C₁-C₆-alkylthio and C₁-C₆-alkylcarbonyl ;
R^{7b}, R^{7c} R^{7d}, R^{7e} R^{7f} R^{8b} R^{8c} R^{8d}, R^{8e} R^{8f} independently from each other, represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7c}, -NR^{7c}R^{8c}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
R^{7b} and R^{8b}, R^{7c} and R^{8c}, R^{7e} and R^{8e}, and R^{7f} and R^{8f} independently from each other, together with the nitrogen atom to which they are attached in groups -NR^{7b}R^{8b}, -NR^{7c}R^{8c}, -NR^{7e}R^{8e}, and - NR^{7f}R^{8f} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂. group, and can optionally contain one or more double bonds ;
R⁹, R^{9a}, independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)q'-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
R⁹ and R^{9a}, together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
L is selected from the group comprising, preferably consisting of C₁-C₆-alkylene or C₃-C₁₀-cycloalkylene ;
X is selected from the group comprising, preferably consisting of, halogen, nitro, cyano, -(CH₂)ᵣOR^{7e}, -(CH₂)ᵣNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7e}, -(CH₂)ᵣC(O)NR^{7e}R^{8e} and - (CH₂)ᵣS(O)₂NR^{7e}R^{8e} ;
Y is selected from the group comprising, preferably consisting of, hydrogen, halogen, nitro, cyano, -(CH₂)ₛOR^{7f}, -(CH₂)ₛNR^{7f}R^{8f}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7f}, -(CH₂)ₛC(O)NR^{7f}R^{8f} and - (CH₂)ₛS(O)₂NR^{7f}R^{8f} ;
m represents an integer of 3 ; and
n, q, q', r, s independently from each other represent an integer of 0, 1, or 2.

3. The compound of general formula (I) according to claim 1 or 2, wherein :
A is meta-phenylene ;
B is phenylene ;
C is arylene or heteroarylene ;
Z is NR³ ;
R¹, R², R³ and R⁵ are hydrogen ;
R⁴ is hydrogen or C₁-C₆-alkyl;
R⁶ is selected from the group comprising, preferably consisting of, hydrogen, halogen, methyl, -OH ;
R^{7c}, R^{7d}, R^{7e}, R^{7f}, R^{8c} R^{8d} R^{8e}, R^{8f} independently from each other, represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7c}, -NR^{7c}R^{8c}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a} ; or
R^{7c} and R^{8c}, R^{7e} and R^{8e}, and R^{7f} and R^{8f} independently from each other, together with the nitrogen atom to which they are attached in groups -NR^{7c}R^{8c}, -NR^{7e}R^{8e}, and -NR^{7f}R^{8f} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
R⁹, R^{9a}, independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
R⁹ and R^{9a}, together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
L is selected from the group comprising, preferably consisting of C₁-C₆-alkylene or C₃-C₁₀-cycloalkylene ;
X is selected from the group comprising, preferably consisting of, halogen, nitro, cyano, -(CH₂)ᵣOR^{7e}, -(CH₂)ᵣNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7e}, -(CH₂)ᵣC(O)NR^{7e}R^{8e} and - (CH₂)ᵣS(O)₂NR^{7e}R^{8e};
Y is selected from the group comprising, preferably consisting of, hydrogen, halogen, nitro, cyano, -(CH₂)ₛOR^{7f}, -(CH₂)ₛNR^{7f}R^{8f}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7f}, -(CH₂)ₛC(O)NR^{7f}R^{8f} and - (CH₂)ₛS(O)₂NR^{7f}R^{8f} ;
m represents an integer of 3 ; and
n, q, q', r, s independently from each other represent an integer of 0, 1, or 2 .

4. The compound of general formula (I) according to any one of claims 1 to 3, wherein :
A is meta-phenylene ;
B is para-phenylene ;
C is phenylene ;
Z is NR³ ;
R¹, R², R³, R⁴, and R⁵ are hydrogen ;
R⁶ is selected from the group comprising, preferably consisting of, hydrogen, fluoro ;
R^{7c}, R^{7d}, R^{7e}, R^{7f}, R^{8c}, R^{8e} independently from each other, represent hydrogen, or are selected from the group comprising, preferably consisting of, C₁-C₆-alkyl, C₃-C₆-cycloalkyl, wherein said residues are unsubstituted or substituted one or more times independently from each other with -OR^{7c} or - NR^{7c}R^{8c} ; or
R^{7c} and R^{8c}, and R^{7e} and R^{8e} independently from each other, together with the nitrogen atom to which they are attached in groups -NR^{7c}R^{8c}, -NR^{7e}R^{8e}, and form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d} or oxygen ;
L is selected from the group comprising, preferably consisting of C₁-C₃-alkylene or C₃-C₆-cycloalkylene ;
X is selected from the group comprising, preferably consisting of, halogen, -(CH₂)ᵣOR^{7e}, -(CH₂)ᵣNR^{7e}R^{8e}, C₁-C₆-alkyl, C₁-C₆-haloalkyl, C₁-C₆-haloalkoxy ;
Y is selected from the group comprising, preferably consisting of, hydrogen, halogen, -(CH₂)ₛOR^{7f}, C₁-C₆-alkyl, C₁-C₆-haloalkyl ;
m represents an integer of 3 ;
n and r independently from each other represent an integer of 0 or 1 ; and
s represents an integer of 0.

5. The compound of general formula (I) according to any one of claims 1 to 4, wherein :
A is meta-phenylene ;
B is para-phenylene ;
C is phenylene ;
Z is N_{R}³;
R¹, R², R³, R⁴ R⁵ , and R⁶ is hydrogen ;
R^{7c}, R^{7d}, R^{7e}, R^{7f} , R^{8c} R^{8e} independently from each other, represent hydrogen, or C₁-C₆-alkyl, wherein said C₁-C₆-alkyl residues are unsubstituted or substituted one or more times independently from each other with -OR^{7c} or -NR^{7c}R^{8c} ; or
R^{7c} and R^{8c}, and R^{7e} and R^{8e} independently from each other, together with the nitrogen atom to which they are attached in groups -NR^{7c}R^{8c}, -NR^{7e}R^{8e}, and form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d} or oxygen ;
L is selected from the group comprising, preferably consisting of C₁-C₃-alkylene or C₃-cycloalkylene ;
X is selected from the group comprising, preferably consisting of, halogen, -(CH₂)ᵣOR^{7e}, -(CH₂)ᵣNR^{7e}R^{8e}, C₁-C₆-alkyl, C₁-C₆-haloalkyl, C₁-C₆-haloalkoxy ;
Y is selected from the group comprising, preferably consisting of, hydrogen, halogen, -(CH₂)ₛOR^{7f}, C₁-C₆-alkyl, C₁-C₆-haloalkyl ;
m represents an integer of 3 ;
n represents an integer of 0 ;
r represents an integer of 0 or 1 ; and
s represents an integer of 0.

6. The compound of general formula (I) according to any one of claims 1 to 5, which is selected from the group consisting of :
1-p-Tolyl-cyclopropane-carboxylic acid [4-(13,13-dioxo-13λ⁶-thia-2,4,8,12,19-pentaaza-tricyclo[12.3.1.1³,⁷]nonadeca-1 (18),3(19),4,6,14,16-hexaen-6-yl)-phenyl]-amide ;
1-o-Tolyl-cyclopropane-arboxylic acid [4-(13,13-dioxo- 13λ⁶-thia-2,4,8,12,19-pentaaza-tricyclo[12. 3.1.1^{3,7}]nonadeca-1 (18),3(19),4,6,14,16-hexaen-6-yl)-phenyl]-amide ;
1-(3-Methoxy-phenyl)-cyclopropane-carboxylic acid [4-(13,13-dioxo-13λ⁶ -thia-2,4,8,12,19-pentaaza-tricyclo[12.3.1.1^{3,7}]nonadeca-1(18),3(19),4,6,14,16-hexaen-6-yl)-phenyl]-amide ;
1-(4-Methoxy-phenyl)-cyclopropane-carboxylic acid [4-(13,13-dioxo-13λ⁶-thia-2,4,8,12,19-pentaaza-tricyclo[12.3.1.1^{3,7}]-nonadeca-1 (18),3(19),4,6,14,16-hexaen-6-yl)-phenyl]-amide ;
1-(3-Chloro-phenyl)-cyclopropane-arboxylic acid [4-(13,13-dioxo-13λ⁶-thia-2,4,8,12,19-pentaaza-tricyclo[12.3.1.1^{3,7}-nadeca-1 (18),3(19),4,6,14,16-hexaen-6-yl)-phenyl]-amide ;
1-(4-Chloro-phenyl)-cyclopropane-carboxylic acid [4-(13,13-dioxo-13λ⁶-thia-2,4,8,12,19-pentaaza-tricyclo[12.3.1.1^{3,7}]-nonadeca-1 (18),3(19),4,6,14,16-hexaen-6-yl)-phenyl]-amide ;
1-(2-Fluoro-phenyl)-cyclopropane-carboxylic acid [4-(13,13-dioxo-13λ⁶-thia-2,4,8,12,19-pentaaza-tricyclo[12.3.1.1^{3,7}]nonadeca-1 (18),3(19),4,6,14,16-hexaen-6-yl)-phenyl]-amide ;
1-(3-Fluoro-phenyl)-cyclopropane-carboxylic acid [4-(13,13-dioxo-13λ⁶-thia-2,4,8,12,19-pentaaza-tricyclo[12.3.1.1^{3,7}]nonadeca-1 (18),3(19),4,6,14,16-hexaen-6-yl)-phenyl]-amide ;
1-(4-Ftuoro-phenyl)-cyclopropane-carboxylic acid [4-(13,13-dioxo-13λ⁶-thia-2,4,8,12,19-pentaaza-tricyclo[12.3.1.1^{3,7}]nonadeca-1 (18),3(19),4,6,14,16-hexaen-6-yl)-phenyl]-amide ;
1-(3-Trifluoromethyl-phenyl)-cyclopropanecarboxylic acid [4-(13,13-dioxo-13λ⁶-thia-2,4,8,12,19-pentaaza-tricyclo[12.3.1.1^{3,7}]nonadeca-1 (18),3(19),4,6,14,16-hexaen-6-yl)-phenyl]-amide ;
1-(4-Trifluoromethyl-phenyl)-cyclopropane-carboxylic acid [4-(13,13-dioxo-13λ⁶-thia-2,4,8,12,19-pentaaza-tricyclo[12.3.1.1^{3,7}]nonadeca-1 (18),3(19),4,6,14,16-hexaen-6-yl)-phenyl]-amide ;
1-(2-Fluoro-5-trifluoromethyl-phenyl)-cyclopropane-carboxylic acid [4-(13,13-dioxo-13λ⁶-thia-2,4,8,12,19-pentaaza-tricyclo[12.3.1.1^{3,7}]-nonadeca-1(18),3(19),4,6,14,16-hexaen-6-yl)-phenyl]-amide ;
2-(4-Chloro-phenyl)-N-[4-(13,13-dioxo-13λ⁶-thia-2,4,8,12,19-pentaaza-tricyclo[12.3.1.1^{3,7}]-nonadeca-1 (18),3(19),4,6,14,16-hexaen-6-yl)-phenyl]-isobutyramide.

7. A method of preparing a compound of general formula (I) according to any one of claims 1 to 6, said method comprising the step of allowing an intermediate compound of general formula A : 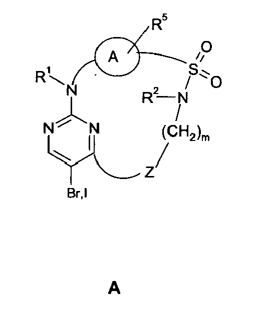 in which the meanings of R¹, R², R⁵, A, Z, and m are as defined in claim 1,
to react with an intermediate compound of general formula B : 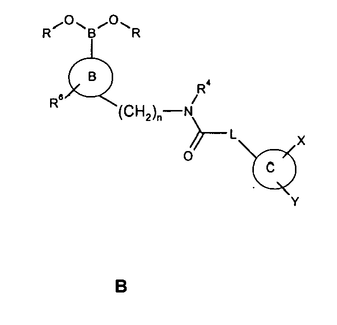 in which R⁴, R⁶, B, C, L, X, Y, and n are as defined in claim 1, and R is either H or two R residues may form a cyclic boronic ester, e.g. a pinacolate ester,
thus providing a compound of general formula (I) : 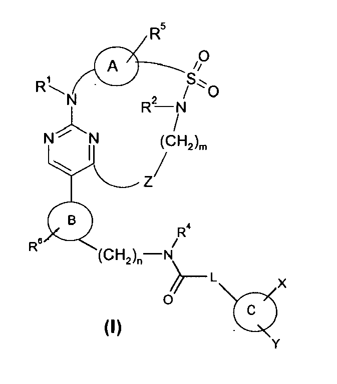 in which R¹, R², R⁴, R⁵, R⁶, A, B, C, L, X, Y, Z, n, and m are as defined in claim 1.

8. A pharmaceutical composition which comprises a compound of general formula (I) according to any one of claims 1 to 6, or a pharmaceutically acceptable salt or an *in vivo* hydrolysable ester thereof, and a pharmaceutically-acceptable diluent or carrier.

9. Use of a compound of any one of claims 1 to 6 for manufacturing a pharmaceutical composition for the treatment of diseases of dysregulated vascular growth or of diseases which are accompanied with dysregulated vascular growth.

10. Use according to claim 9, wherein said diseases are tumours and/or metastases thereof.

11. Use according to claim 9, wherein said diseases are retinopathy, other angiogenesis dependent diseases of the eye, rheumatoid arthritis, and other inflammatory diseases associated with angiogenesis.

12. Use of claim 11, wherein said angiogenesis dependent diseases of the eye are cornea transplant rejection, age-related macular degeneration.

13. Use according to claim 9, wherein said diseases are coronary and peripheral artery disease.

14. Use of claim 11, wherein said inflammatory diseases associated with angiogenesis are psoriasis, delayed type hypersensitivity, contact dermatitis, asthma, multiple sclerosis, restenosis, pulmonary hypertension, stroke, and diseases of the bowel.

15. Use according to claim 9, wherein said diseases are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

16. A method of treating a disease of dysregulated vascular growth or diseases which are accompanied with dysregulated vascular growth by administering an effective amount of a compound of general formula (I) according to any one of claims 1 to 6.

17. The method according to claim 16, wherein said diseases are tumours and/or metastases thereof.

18. The method according to claim 16, wherein said diseases are retinopathy, other angiogenesis dependent diseases of the eye, rheumatoid arthritis, and other inflammatory diseases associated with angiogenesis.

19. The method according to claim 18, wherein said angiogenesis dependent diseases of the eye are cornea transplant rejection, age-related macular degeneration.

20. The method according to claim 18, wherein said diseases are coronary and peripheral artery disease.

21. The method according to claim 18 wherein said inflammatory diseases associated with angiogenesis are psoriasis, delayed type hypersensitivity, contact dermatitis, asthma, multiple sclerosis, restenosis, pulmonary hypertension, stroke, and diseases of the bowel.

22. The method according to claim 16, wherein said diseases are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

23. Use of a compound of general formula A : 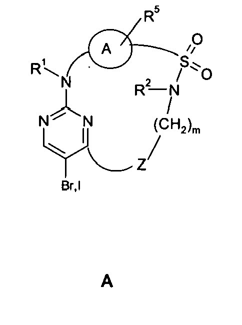 in which the meanings of R¹, R², R⁵, A, Z, and m are as defined in claim 1, for the preparation of a compound of general formula (I) : 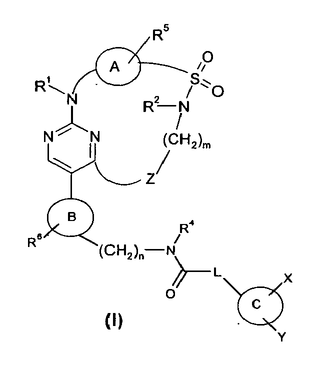 in which R¹, R², R⁴, R⁵, R⁶, A, B, C, L, X, Y, Z, n, and m are as defined in claim 1.

24. Use of a compound of general formula B : 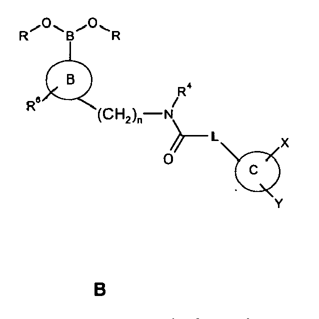 in which R⁴, R⁶, B, C, L, X, Y, and n are as defined in claim 1, and R is either H or two R residues may form a cyclic boronic ester, e.g. a pinacolate ester, for the preparation of a compound of general formula (I) : 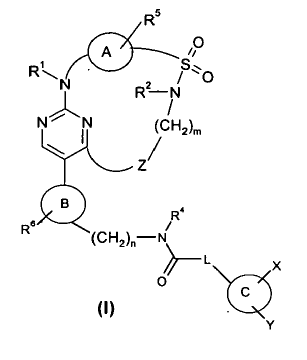 in which R¹, R², R⁴, R⁵, R⁶, A, B, C, L, X, Y, Z, n, and m are as defined in claim 1.
